(19) Europäisches Patentamt
European Patent Office
Office européen des brevets

(11) EP 2 257 807 B1

(12) FASCICULE DE BREVET EUROPEEN

(45) Date de publication et mention de la délivrance du brevet:
**19.12.2018 Bulletin 2018/51**

(21) Numéro de dépôt: **09719721.4**

(22) Date de dépôt: **05.03.2009**

(51) Int Cl.:
*G01N 33/543* $^{(2006.01)}$

(86) Numéro de dépôt international:
**PCT/EP2009/052637**

(87) Numéro de publication internationale:
**WO 2009/112430 (17.09.2009 Gazette 2009/38)**

(54) **PROCÉDÉ DE DÉTECTION ET DE QUANTIFICATION D'UNE MOLÉCULE COMPRENANT AU MOINS UN GROUPEMENT PROTONÉ SUR UN SUPPORT SOLIDE**

VERFAHREN ZUM NACHWEIS UND ZUR QUANTIFIZIERUNG EINES WENIGSTENS EINE PROTONGRUPPE ENTHALTENDEN MOLEKÜLS AUF EINEM FESTEN SUBSTRAT

METHOD FOR DETECTING AND QUANTIFYING A MOLECULE INCLUDING AT LEAST ONE PROTON GROUP ON A SOLID SUBSTRATE

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **05.03.2008 FR 0851425**

(43) Date de publication de la demande:
**08.12.2010 Bulletin 2010/49**

(73) Titulaire: **Centre National d'Etudes Spatiales 75001 Paris (FR)**

(72) Inventeurs:
• **COUSSOT, Gaëlle**
  **F-34400 Lunel (FR)**
• **VANDENABEELE-TRAMBOUZE, Odile**
  **29400 Locmelar (FR)**
• **DESVIGNES, Isabelle**
  **F-34990 Juvignac (FR)**
• **DOBRIJEVIC, Michel**
  **F-33600 Pessac (FR)**
• **LE POSTOLLEC, Aurélie**
  **F-33670 Creon (FR)**
• **CHAZALNOEL, Pascale**
  **F-31280 Dremil-Lafage (FR)**

(74) Mandataire: **Gaillarde, Frédéric F. Ch. et al Cabinet Germain & Maureau 31-33, rue de la Baume 75008 Paris (FR)**

(56) Documents cités:
• ZHU Y ET AL.: "Density quantification of collagen grafted on biodegradable polyester: Its application to esophageal smooth muscle cell" ANALYTICAL BIOCHEMISTRY, vol. 363, no. 1, avril 2007 (2007-04), pages 119-127, XP005911000 ISSN: 0003-2697
• KETT W C ET AL.: "Direct detection of the binding of avidin and lactoferrin fluorescent probes to heparinized surfaces" ANALYTICAL BIOCHEMISTRY, vol. 339, no. 2, avril 2005 (2005-04), pages 206-215, XP005197352 ISSN: 0003-2697
• GOSNELL M C ET AL.: "Determination of Activity and Amount of Silica-Immobilized Penicillinase" MICROCHEMICAL JOURNAL, vol. 37, no. 2, avril 1988 (1988-04), pages 149-154, XP009107316 ISSN: 0026-265X
• ORSCHEL M ET AL.: "Evaluation of several methods to quantify immobilized proteins on gold and silica surfaces" COLLOIDS AND SURFACES B: BIOINTERFACES, vol. 10, no. 5, 15 avril 1998 (1998-04-15), pages 273-279, XP009107000 NL ISSN: 0927-7765
• BONDE M ET AL.: "Direct dye binding--a quantitative assay for solid-phase immobilized protein." ANALYTICAL BIOCHEMISTRY, vol. 200, no. 1, janvier 1992 (1992-01), pages 195-198, XP009107228 ISSN: 0003-2697

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- LU B ET AL.: "A Planar Quartz Waveguide Immunosensor Based on TIRF Principle" ANALYTICAL LETTERS, vol. 25, no. 1, janvier 1992 (1992-01), pages 1-10, XP009107113 ISSN: 0003-2719
- NEUHOFF V ET AL.: "Clear background and highly sensitive protein staining with Coomassie Blue dyes in polyacrylamide gels: A systematic analysis" ELECTROPHORESIS, vol. 6, no. 9, 1985, pages 427-448, XP009107061 ISSN: 0173-0835
- MARKO I E ET AL.: "Efficient and convergent stereocontrolled spiroannulation of ketones." TETRAHEDRON LETTERS, vol. 44, no. 16, 14 avril 2003 (2003-04-14), pages 3333-3336, XP004417086 ISSN: 0040-4039
- COUSSOT G ET AL.: "Colorimetric quantification of amino groups in linear and dendritic structures" POLYMER INTERNATIONAL, vol. 58, no. 5, 13 mars 2009 (2009-03-13), pages 511-518, XP009117648 JOHN WILEY AND SONS LTD GBR, doi:10.1002/pi.2560

**Description**

**DOMAINE TECHNIQUE**

La présente invention concerne le domaine des supports présentant une interface comprenant au moins un groupement protoné, telle qu'une interface aminée et/ou protéique.

Plus particulièrement, la présente invention vise à fournir un procédé et des moyens capables de caractériser l'interface comprenant au moins un groupement protoné, telle que l'interface aminée et/ou protéique de ces supports. Le procédé selon l'invention est rapide, simple à mettre en oeuvre et réversible. Cette réversibilité permet une utilisation de l'interface après la mise en oeuvre du procédé de l'invention. Il présente une sensibilité et une linéarité de dosage remarquables, sans affecter l'interface ce qui permet l'utilisation éventuelle post-caractérisation des supports ainsi recouverts.

**ÉTAT DE LA TECHNIQUE ANTÉRIEURE**

Il existe différents types de supports présentant à leur surface des groupements protonés, tels que des alkyles amines ou autres groupements aminés et notamment des peptides, des polypeptides ou des protéines dans le domaine des réactifs utiles en biologie et, plus particulièrement, dans les domaines de la culture cellulaire, du diagnostic et des analyses biologiques.

En effet, les supports présentant, à leur surface, une matrice constituée d'une (ou plusieurs) protéine(s) sont des supports notamment utilisés dans le cadre de la culture des cellules puisqu'ils favorisent l'adhésion des cultures cellulaires et, éventuellement, leur différenciation. De tels supports sont également connus sous le terme de « supports coatés ».

Les supports du type puces à peptides, puces à protéines, puces à anticorps ou puces à cellules sont également des outils largement utilisés tant en recherche fondamentale qu'en recherche clinique. En effet, de tels supports sont utilisés pour évaluer une réponse immunitaire et, notamment, pour rechercher des anticorps spécifiques d'épitopes ou de mimotopes fixés sur ces derniers. La détection d'interactions moléculaires met également en oeuvre des supports de type puces à peptides / à protéines / à anticorps / à cellules.

Lors de la préparation ou lors de l'utilisation de ces supports présentant à leur surface des groupements aminés, il est nécessaire de vérifier le greffage des fonctions amines dans un souci de vérification dudit greffage, de son homogénéité ou de sa stabilité.

Le brevet US 6,696,304 propose un procédé pour déterminer la quantité moyenne relative d'une molécule comprenant au moins une amine, immobilisée sur un support solide. Ce procédé met en oeuvre (1) un (ou plusieurs) marqueur(s) émettant de la lumière et (2) des supports solides sur lesquels sont immobilisées des quantités connues de molécule(s) contenant une (ou plusieurs) amine(s), lesdits supports solides servant de références. Le procédé objet du brevet US 6,696,304 est, d'une part, irréversible comme indiqué colonne 10, lignes 29 à 32 et, d'autre part, lourd à mettre en oeuvre puisqu'il nécessite l'utilisation de standards.

L'homme du métier connaît différentes techniques pour quantifier les protéines en solution comme la méthode de Lowry, la méthode de Bradford, la méthode de Biuret, etc....

Le bleu de Coomassie est l'agent de coloration utilisé dans la méthode de Bradford pour estimer en solution, rapidement i.e. dans un temps inférieur à 5 min, la concentration protéique d'un milieu. La méthode de Bradford permet de réaliser des dosages de protéines en solution avec des seuils de détection de l'ordre du $\mu$g de protéines par mL de solution. Dans les conditions décrites par Bradford [Bradford, 1976] mettant en oeuvre une solution dont le pH est inférieur à 1, le complexe bleu-protéine formé entraîne un transfert du pic d'absorption qui passe du rouge ($\lambda_{max}$ = 470 nm, pH < 1) (forme cationique libre) au vert ($\lambda_{max}$ = 650 nm, pH ~ 1) (forme neutre) puis bleu ($\lambda_{max}$ = 595 nm, $2 \leq$ pH $\leq$ 11) (forme liée anionique) . Il existe, pour le bleu de Coomassie, une 4$^{ième}$ forme de couleur "violet-rose" ($\lambda_{max}$ = 530 nm, pH $\geq$ 11) peu évoquée jusqu'à présent dans la littérature. Seule une référence bibliographique datant de 1993 y fait référence [Chial, 1993]. De plus, aucun dosage en solution d'une des 4 formes libres du bleu de Coomassie ($\lambda_{max}$ = 470, 650, 595 ou 530) n'a également été reporté dans la littérature jusqu'à ce jour.

Le brevet US 6,555,382 décrit un procédé de coloration utilisant le bleu de Coomassie pour visualiser de façon rapide des protéines présentes dans un gel, sur une membrane ou autre support poreux. Ce procédé de coloration met en oeuvre une solution contenant un acide dilué à une concentration de 1 à 100 mM et du bleu de Coomassie à une concentration de 0,0005 à 0,5 % (p/v). Cependant, l'invention décrite dans le brevet US 6,555,382 ne permet ni une estimation directe de la quantité de protéine présente par bande, ni une analyse quantitative du nombre d'amines présentes sur la protéine « colorée », la possibilité de décrocher l'agent de coloration n'ayant pas été évoquée.

Gosnell et al, 1988 (Microchemical Journal, vol. 37, pages 149-154) propose une méthode de quantification de la pénicillinase immobilisée sur un support solide. Le support est mis en présence de 0,1% en masse de bleu de Coomassie G250 pendant 30 min, puis lavé avec une solution acide acétique/isopropanol/H$_2$O (10/25/65; v/v/v). L'agent de coloration est décroché en présence de NaOH (0,1 M) contenant 80% de méthanol. Après acidification, l'absorbance de la solution de décrochage contenant l'agent de coloration est mesurée.

Dans Orschel et al, 1998 (Colloids and Surfaces B : Biointerfaces, vol. 10, pages 273-279), l'immobilisation covalente du complexe streptavidine-biotine est réalisée sur des surfaces de silice et d'or. Cinq méthodes sont décrites et comparées pour déterminer la quantité de complexes streptavidine-biotine immobilisée. Parmi ces dernières, se trouve la méthode photométrique décrite par Gosnell et al, 1988.

Il n'existe pas de procédé colorimétrique simple, sensible (< 1 $\mu$g de protéine) et réversible permettant de mettre en évidence et/ou de quantifier la présence de sites protonés appartenant par exemple à des protéines, de peptides ou de toute molécule comprenant au moins une amine, greffés sur un support solide (poreux ou non) et permettant l'utilisation ultérieure de ce support (i.e. après la mise en évidence et/ou la quantification).

## EXPOSÉ DE L'INVENTION

La présente invention permet de résoudre le problème technique ci-dessus en proposant une nouvelle stratégie analytique basée sur le dosage direct d'un agent de coloration. Par « dosage direct », on entend le dosage de l'agent de coloration « dégreffé » du support à caractériser après avoir fixé cet agent colorimétrique sélectivement sur l'amine présente à la surface du support puis éliminer l'excédent de l'agent de coloration. La présente invention vise à caractériser des supports aminés (bruts ou « coatés » c'est-à-dire des supports recouverts d'une interface). Par « caractérisation » dans le cadre de la présente invention, on entend non seulement la détection d'amine(s) éventuellement présente(s) à la surface d'un support solide mais aussi leur quantification i.e. la détermination du nombre d'amines disponibles en surface de ce support solide. Le nombre de fonctions amines sera exprimé par unité de surface ou par unité de masse du support.

La présente invention définie dans le jeu de revendications annexée est remarquable de par le fait qu'elle s'applique non seulement au domaine des supports coatés, des puces à peptides, à protéines, à anticorps ou à cellules mais aussi à tout domaine utilisant un support solide présentant à sa surface au moins un groupement protoné tel qu'une fonction amine.

La présente invention concerne un procédé de détection et de quantification d'au moins une molécule comprenant au moins un groupement protoné tel qu'une fonction amine immobilisée à la surface d'un support solide, ledit procédé étant un procédé direct, colorimétrique et mettant en oeuvre un agent de coloration.

Plus particulièrement, la présente invention concerne un procédé de détection et de quantification d'au moins une molécule comprenant au moins un groupement protoné tel qu'une fonction amine immobilisée à la surface d'un support solide, comprenant les étapes successives suivantes :

a) mise en contact de ladite surface du support solide sur laquelle est immobilisé au moins une molécule comprenant au moins un groupement protoné avec une solution $T_1$ contenant du bleu de Coomassie G250 comme agent de coloration,
ledit groupement protoné étant choisi dans le groupe constitué par une fonction amine, une fonction imine, une fonction guanidino et un groupement hétéroaryle,
b) élimination dudit agent de coloration n'ayant pas réagi avec ladite surface lors de l'étape (a),
c) mise en contact de ladite surface avec une solution $T_2$ apte à dissocier l'éventuel (ou les éventuels) complexe(s) formé(s) entre ledit agent de coloration et ladite molécule comprenant au moins un groupement protoné tel qu'une fonction amine,
ladite solution $T_2$ étant une solution aqueuse contenant un alcool dans une proportion comprise entre 40% et 60% en volume par rapport au volume total de la solution $T_2$ et des ions carbonates et le pH de ladite solution $T_2$ étant supérieur à 11,
d) acidification de ladite solution $T_2$ et détection dudit agent de coloration éventuellement présent dans la solution $T_2$ de façon à détecter et quantifier ladite molécule comprenant au moins un groupement protoné tel qu'une fonction amine,

ladite détection de l'agent de coloration lors de l'étape (d) consistant à mesurer la densité optique de la solution T2 acidifiée obtenue après l'étape (c),
ladite détection et ladite quantification de ladite molécule comprenant au moins un groupement protoné étant obtenues à partir de la densité optique réelle correspondant à la densité optique de la solution T2 acidifiée obtenue après l'étape (c) à laquelle a été soustraite la densité optique obtenue, dans les mêmes conditions mais en absence de ladite au moins -une molécule comprenant au moins un groupement protoné.

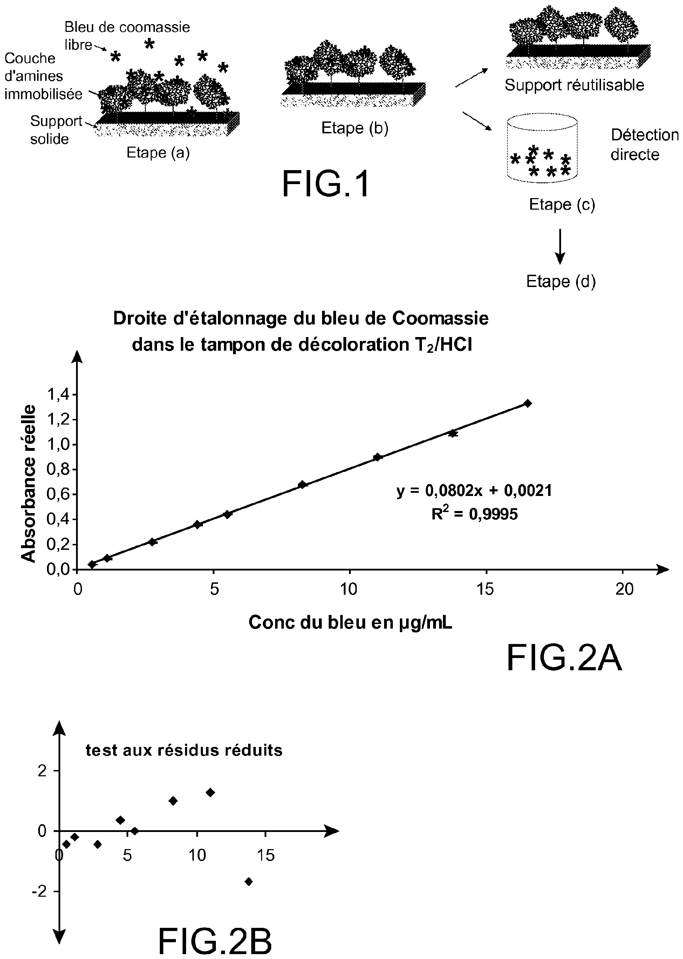

Les principales étapes du procédé selon l'invention sont schématisées sur la figure 1 avec, comme agent de coloration, du bleu de Coomassie.

Par « agent de coloration », on entend, dans le cadre de la présente invention, un composé chimique présentant des propriétés d'absorption ou d'émission dans le domaine de la lumière visible. Par extension, on pourra également désigner par « agent de coloration » un composé présentant des propriétés d'absorption, d'émission ou de réémission dans un domaine plus large, allant du proche ultra-violet au proche infrarouge, c'est-à-dire dans une gamme de longueurs d'onde allant généralement de 280 à 3000 nm. De plus, l'agent de coloration mis en oeuvre dans le cadre de la présente invention est un colorant organique capable de réagir avec une molécule comprenant au moins un groupement protoné tel qu'une fonction amine.

Les agents de coloration connus dans l'état de la technique sont notamment choisis dans les familles des anthraquinones, des mono- et dichlorotriazines, des dérivés (di)azo et des triphénylméthanes. Ces familles comprennent, entre autre, le Rouge Ponceau (ou Ponceau S) (CAS No. 6226-79-5), le Ponceau 2R (CAS No. 3761-53-3), Ponceau 4R (CAS No. 2611-82-7), Ponceau 6R (CAS No. 2766-77-0), l'azorubine (CAS No. 3567-69-9), le Procion Yellow 4R, le Procion rubine (CAS No. 17752-85-1), le Brilliant Blue FCF (CAS No. 3844-45-9), le Remazol Brilliant Blue (CAS No. 2580-78-1), le Fast Green FCF (CAS No. 2353-45-9), le Noir Amido (CAS No. 1064-48-8), le bleu de Procion (CAS No. 12236-82-7), le Serva Violet (CAS No. 910010-03-9), le Bleu de Coomassie (CAS No. 78642-64-5 ou CAS No. 6104-58-1) et leurs mélanges

L'agent de coloration connu dans l'état de la technique peut également être sélectionné parmi les agents fluorescents ou les agents phosphorescents. Ces agents fluorescents ou ces agents phosphorescents sont avantageusement des agents fluorescents ou des agents phosphorescents présentant au moins une fonction sulfonate et/ou au moins une fonction carboxylate. A titre d'exemples et de façon non exhaustive, on peut citer, parmi de tels agents fluorescents ou phosphorescents, la fluorescéine et ses dérivés tels que la 6-carboxy-fluorescéine (CAS No. 3301-79-9) ; l'éosine Y (CAS No. 17372-87-1) ; l'éosine B (CAS No. 548-28-3) ; la rhodamine B (CAS No. 81-88-9), la sulforhodamine 101 (CAS No. 60311-02-6), la pyranine (CAS No. 6358-69-6) et la cardiogreen (Cas No 3599-32-4), etc...

L'agent de coloration mis en oeuvre dans le cadre de la présente invention est le Bleu de Coomassie G250.

Le groupement protoné mis en oeuvre dans le cadre de la présente invention est un groupe portant une charge électrique élémentaire positive, cette dernière étant portée par un atome d'azote. Aussi, le groupement protoné est choisi dans le groupe constitué par une fonction amine, une fonction imine, une fonction guanidino et un groupement hétéroaryle.

Par « groupement hétéroaryle », on entend, dans le cadre de la présente invention, une structure carbonée hétéroaromatique, éventuellement mono- ou polysubstituée, constituée d'un ou plusieurs cycles hétéroaromatiques comportant chacun de 3 à 8 atomes, ladite structure présentant un hétéroatome N, le ou les autres éventuels hétéroatomes pouvant être N, O, P ou S. Le ou les substituants peuvent contenir un ou plusieurs hétéroatomes, tels que N, O, F, Cl, P, Si, Br ou S ainsi que des groupes alkyles notamment en $C_1$ à $C_6$.

La fonction amine susceptible d'être mise en oeuvre dans le cadre de la présente invention est une fonction amine primaire du type ($-NH_2$), une fonction amine secondaire du type (-NHR, R représentant un groupe carboné), une fonction amine tertiaire du type (-NR'R, R et R' représentant des groupes carbonés identiques ou différents, ou appartenant à un même groupe carboné (cas des amines cycliques)). De façon préférée, la fonction amine mise en oeuvre dans le cadre de la présente invention est une fonction amine primaire du type ($-NH_2$), une fonction amine secondaire du type (-NHR, R représentant un groupe carboné).

La molécule comprenant au moins un groupement protoné tel qu'une fonction amine susceptible d'être utilisée dans le cadre de la présente invention est n'importe quelle molécule présentant au moins un groupement protoné tel qu'une fonction amine connue de l'homme du métier. Cette molécule est notamment choisie dans le groupe constitué par les peptides, les polypeptides, les protéines, les protéines présentant des groupes prosthétiques (telles que glycoprotéines, lipoprotéines, nucléoprotéines, métalloprotéines, chromoprotéines, hémoprotéines), les anticorps, les acides nucléiques transaminés, les amines biogènes, les alkylamines, les polymères aminés, les dendrons et les dendrimères tels que les structures dendrimériques de type PAMAM (PolyAMidoAMine) ou de type PPI (Poly(PropyleneImine)) ou les structures dendrimériques décrites dans la demande internationale WO 2006/114528), leurs fragments et leurs dérivés.

A titre d'exemples de protéines susceptibles d'être mises en oeuvre dans le cadre de la présente invention et déjà utilisées comme protéines d'interface de surfaces coatées, on peut citer la streptavidine, la gélatine, le collagène et notamment le collagène de type 1, la fibronectine, le lysozyme, la polylysine linéaire, la sérum albumine bovine, etc.... Par ailleurs, des polymères aminés de type polyacrylamide et notamment tels que les poly(éthyleneimine) (PEI) de formule $-(CH_2-CH_2-NH)_n-$ avec $10<n<10^5$ ont également été utilisés comme protéines d'interface de surfaces coatées.

La présente invention envisage d'utiliser des dérivés des molécules contenant un groupement protoné tel qu'une fonction amine définies ci-dessus. Par « dérivé », on entend des molécules qui présentent 60%, 65%, 70%, 75%, 80%, 85%, 90% et/ou 95% d'identité avec les molécules comprenant au moins un groupement protoné tel qu'une fonction amine définies ci-dessus et notamment vis-à-vis des séquences de nucléotides ou des séquences d'acides aminés de ces molécules.

Lorsque les molécules comprenant au moins un groupement protoné tel qu'une fonction amine présentent un (ou plusieurs) acide(s) aminé(s), leurs dérivés peuvent présenter au moins un acide aminé supplémentaire, une modification post-traductionelle et/ou une modification chimique (en particulier une glycosylation, une amidation, une acylation, une acétylation, une méthylation), ou un groupement protecteur qui permet d'éviter leur dégradation.

Les dérivés des molécules contenant un groupement protoné tel qu'une fonction amine et présentant un (ou plusieurs) acide(s) aminé(s) peuvent également être ceux dont un (ou plusieurs) acide (s) aminé(s) est (sont) choisi (s) dans le groupe constitué par des énantiomères, des diastéréoisomères, des acides aminés naturels de conformation D, des bêta acides aminés, des acides aminés alpha substitués, des acides aminés rares notamment l'hydroxyproline, l'hydroxylysine, l'allo-hydroxylysine, la 6-N-méthyllysine, la N-éthylglycine, la N-méthylglycine, la N-éthylasparagine, l'allo-isoleucine, la N-méthylisoleucine, la N-méthylvaline, la pyroglutamine, l'acide aminobutyrique et des acides aminés synthétiques notamment l'ornithine, la norleucine, la norvaline, la cyclohéxyl-alanine et les oméga-acides aminés.

Les fragments des molécules comprenant au moins un groupement protoné tel qu'une fonction amine sont, à titre d'exemples et de façon non exhaustive :

- des fragments peptidiques présentant avantageusement plus de 8 acides aminés, notamment plus de 10 acides aminés ou encore plus de 15 acides aminés,
- des fragments d'anticorps comportant au moins le domaine variable d'une chaîne lourde (VH) et/ou le domaine variable d'une chaîne légère (VL) d'une immunoglobuline classique, ou bien le domaine variable d'une immunoglobuline à chaîne unique, tel que les fragments Fab, Fv, scFv ou VHH,
- des fragments d'acides nucléiques transaminés présentant avantageusement plus de 5 nucléotides, notamment plus de 10 nucléotides ou encore plus de 15 nucléotides.

Les molécules contenant un groupement protoné tel qu'une fonction amine, leurs dérivés et leurs fragments tels que précédemment définis peuvent être des produits naturels, des produits recombinants obtenus selon des techniques de biologie moléculaire et de génie génétique bien connues de l'homme du métier ou être synthétisés chimiquement selon des techniques telles que la synthèse en phase solide ou liquide également bien connues de l'homme du métier.

Dans le cadre de la présente invention, tout support solide à la surface duquel une (ou plusieurs) molécule(s) comprenant au moins un groupement protoné tel qu'une fonction amine peu(ven)t être immobilisée(s) est susceptible d'être utilisé dans le cadre de la présente invention.

Dans une variante de l'invention, le support solide ou du moins la surface dudit support solide où la (ou les) molécule (s) comprenant au moins un groupement protoné tel qu'une fonction amine est (sont) immobilisée(s), est notamment un support solide ou une surface inorganique. En effet, on peut envisager un support solide dont seule la surface est en un matériau inorganique particulier, le reste du support étant dans un autre matériau inorganique ou dans un matériau organique. Avantageusement, le support solide ou la surface dudit support solide est en un matériau inorganique choisi dans le groupe constitué par les verres, les quartz, les céramiques (par exemple, de type oxyde), les métaux (par exemple, aluminium, chrome, cuivre, zinc, argent, nickel, étain ou or), les métalloïdes (par exemple, silicium ou silicium oxydé) et leurs mélanges.

Dans une autre variante de l'invention, le support solide ou du moins la surface dudit support solide où la (ou les) molécule (s) comprenant au moins un groupement protoné tel qu'une fonction amine est (sont) immobilisée(s) est en un matériau organique comme un polymère tel de l'agarose ou une résine incluant le nylon, le polyéthylène glycol, les polycarbonates, les polyfluoropolymères ou les composites. On peut également envisager un support solide dont seule la surface est en un matériau organique particulier, le reste du support étant dans un autre matériau organique ou dans un matériau inorganique.

Ledit support solide peut se présenter sous diverses formes de taille variable. A titre d'exemples et de façon non exhaustive, il peut se présenter sous forme de lames, de microplaques, de particules, de gels tel qu'un gel d'agarose, de billes, de microbilles, de fibres, de tubes tels que des tubes à hémolyse ou de microcanaux de type capillaires. Ces différents types de support peuvent avoir des tailles variant de quelques centaines de micromètres à plusieurs centimètres.

Dans une forme de mise en oeuvre particulière de la présente invention, le support solide présente une surface portant des groupements fonctionnels grâce auxquels la (ou les) molécule(s) comprenant au moins un groupement protoné tel qu'une fonction amine est (sont) capable(s) de s'immobiliser. De façon avantageuse, ces groupements fonctionnels sont choisis parmi les groupements carboxyliques, des entités radicalaires, les fonctions alcools, amines, amides, époxy ou thiols. Cette fonctionnalisation peut être intrinsèque à la nature du matériau en surface du support solide mis en oeuvre. De façon alternative, cette fonctionnalisation peut être obtenue par nettoyage de ladite surface par l'intermédiaire d'au moins un solvant, détergent, rayonnement ou plasma ou tout autre procédé permettant la formation de groupements fonctionnels tels que précédemment définis.

Dans une première variante de la présente invention, la molécule comprenant au moins un groupement protoné tel qu'une fonction amine peut être immobilisée de façon directe à la surface du support solide fonctionnalisé ou non. Un support solide « coaté » avec une protéine est un exemple d'immobilisation directe.

Dans une seconde variante de la présente invention, la molécule comprenant au moins un groupement protoné tel qu'une fonction amine peut être immobilisée de façon indirecte à la surface du support solide fonctionnalisé ou non. Cette immobilisation indirecte fait intervenir un bras espaceur (ou agent de jonction) lié, d'une part, au support solide et, d'autre part, à la molécule comprenant au moins un groupement protoné tel qu'une fonction amine. Le bras espaceur peut ou non réagir avec l'agent de coloration lors de l'étape (a) du procédé selon l'invention et notamment peut ou non présenter un groupement protoné tel qu'une fonction amine susceptible de réagir lors de ladite étape (a). L'homme du métier connaît différents exemples de tels bras espaceurs. De façon non exhaustive, peuvent être cités, en tant que bras espaceurs susceptibles d'être mis en oeuvre dans le cadre de la présente invention, le 1,6 diaminohexane, l'acide 6-aminohexanoïque, un groupe succinimide, un époxyde, l'acide UDP-glucuronique, des chaînes alkyles linéaires ou ramifiées de 1 à 20 atomes de carbone, du polyéthylène glycol, du glutaraldéhyde, etc...

Les liaisons mises en oeuvre au cours d'une immobilisation directe ou indirecte peuvent être toutes liaisons connues de l'homme du métier et notamment des liaisons covalentes, des liaisons ioniques, des liaisons hydrogène, une adsorption, etc...

La première étape du procédé selon l'invention (étape a), dite « de coloration » consiste à mettre en contact la surface susceptible de présenter une molécule comprenant au moins un groupement protoné tel qu'une fonction amine avec une concentration donnée d'agent de coloration. Ledit agent de coloration est dilué dans la solution $T_1$.

La solution $T_1$ est une solution couramment utilisée lors de coloration utilisant un des agents de coloration tels que précédemment définis, notamment lors de dosage en solution, de coloration de gels d'électrophorèse SDS-PAGE ou de coloration de membrane cellulosique. L'homme du métier saura donc en fonction de l'agent de coloration mis en oeuvre quelle solution $T_1$ utiliser (nature et quantité des composants).

Avantageusement, la solution $T_1$ est une solution aqueuse comprenant un alcool et/ou un acide. Le pH de la solution $T_1$ est avantageusement supérieur à 1 et, en particulier, supérieur à 2.

L'alcool contenu dans la solution $T_1$ est avantageusement choisi dans le groupe constitué par le méthanol, l'éthanol, le propanol, l'isopropanol, le butanol et leurs mélanges. La proportion d'alcool est comprise entre 1 et 25 %, notamment entre 2 et 20 % et, en particulier, entre 5 et 15 % en volume par rapport au volume total de solution $T_1$.

L'acide contenu dans la solution $T_1$ est avantageusement choisi dans le groupe constitué par l'acide acétique, l'acide trichloroacétique, l'acide phosphorique, l'acide chlorhydrique, l'acide sulfurique, l'acide perchlorique et leurs mélanges. La proportion d'acide est comprise entre 0,5 et 20 %, notamment entre 1 et 15 % et, en particulier, entre 2 et 10 % en volume par rapport au volume total de solution $T_1$.

L'agent de coloration tel que précédemment défini est utilisé en une quantité comprise entre 0,001 et 1 %, notamment entre 0,005 et 0,5 % et, en particulier, entre 0,01 et 0,1 % en masse par rapport au volume total de solution $T_1$.

L'étape (a) du procédé selon l'invention est avantageusement effectuée à températue ambiante (i.e. température comprise entre 20 et 25°C) et ce sous agitation. La durée de l'étape (a) selon l'invention est supérieure à 3 min, notamment supérieure à 5 min, en particulier, comprise entre 5 min et 2 heures, plus particulièrement, entre 5 min et 1 heure et, tout particulièrement, entre 5 et 30 min.

A titre d'exemple et lorsque l'agent de coloration mis en oeuvre est du Bleu de Coomassie, la solution $T_1$ est composée de 85% d'eau milliQ, 10% de méthanol de qualité analytique et 5% d'acide acétique de qualité analytique et son pH est de 2,2. Le bleu de Coomassie (G-250) est utilisé à 0,05 % en masse dans cette solution lors de l'étape de coloration.

Pour qu'il y ait un maximum d'interactions du Bleu de Coomassie avec la molécule comprenant au moins un groupement protoné tel qu'une amine immobilisée sur un support solide et notamment avec lesdites amines, l'agent de coloration a été utilisé dans la solution $T_1$ à une concentration C très supérieure à celle utilisée lors du dosage en solution par le test de Bradford. Cette concentration C égale ou supérieure à 500 $\mu$g/mL ne permet pas de dosage en solution car il y a alors saturation du signal d'absorbance lors de la lecture directe. Se placer à une concentration de 500 $\mu$g/mL de Bleu de Coomassie permet une réutilisation de la solution $T_1$. En outre, la solution $T_1$ contenant une concentration élevée de Bleu de Coomassie évite des étapes de mise au point selon le format utilisé, elle peut donc être utilisée sur des formats possédant de plus importantes capacités de liaison que les formats de microplaques testées dans la présente invention.

L'étape (b) du procédé selon l'invention vise à éliminer l'agent de coloration n'ayant pas réagi et notamment n'ayant pas formé de complexes avec la molécule comprenant au moins un groupement protoné tel qu'une fonction amine. Cette étape (b) consiste en une étape d'au moins un lavage.

Avantageusement, l'étape (b) du procédé selon l'invention comprend un (ou plusieurs) lavage(s) de la surface du support solide sur laquelle est(sont) immobilisée(s) une (ou plusieurs) molécule(s) comprenant au moins un groupement protoné tel qu'une fonction amine, aux moyens de solutions de lavage identiques ou différentes. En particulier, l'étape (b) du procédé selon l'invention comprend au moins deux lavages, au moins trois lavages, au moins quatre lavages ou au moins cinq lavages de ladite surface du support solide. L'homme du métier saura aisément définir le nombre de lavages nécessaire et suffisant en fonction de l'agent de coloration et de sa concentration utilisés lors de l'étape (a) du procédé et en fonction de la (ou des) solution (s) de lavage employée(s). Ainsi, l'homme du métier pourra récupérer les solutions de lavage et vérifier notamment par une mesure spectrophotométrique la présence dans ces dernières d'agent de coloration. Dès que les solutions de lavage ne contiennent plus d'agent de coloration, l'étape (b) du procédé selon l'invention est achevée.

Les lavages peuvent être effectués avec une solution de lavage identique ou différente. En effet, on peut envisager d'utiliser, lors de l'étape (b), une solution de lavage identique à chaque lavage, des solutions de lavage différentes d'un lavage à l'autre ou des solutions de lavage identiques ou différentes d'un lavage à l'autre. Les lavages peuvent présenter des durées identiques ou différentes ; ces durées s'étendant de quelques minutes (2, 3, 5, 10 ou 15 minutes) à une (ou plusieurs) heure (s) (1, 2, 3, 4, 5, ou 15 heures). L'étape (b) du procédé selon l'invention est avantageusement effectuée sous agitation et à température ambiante mais peut également être effectuée à une température inférieure à la température ambiante et notamment dans une chambre froide (température comprise entre 3 et 6°C).

N'importe quelle solution de lavage connue de l'homme du métier est utilisable dans le cadre de l'étape (b) du procédé selon l'invention. La seule condition est que cette solution de lavage n'affecte ni l'immobilisation de la (ou des) molécule(s) comprenant au moins un groupement protoné tel qu'une fonction amine à la surface du support solide, ni les éventuels complexes formés entre l'agent de coloration mis en oeuvre à l'étape (a) et ladite molécule comprenant au moins un groupement protoné tel qu'une fonction amine.

A titre d'exemples et de façon non exhaustive, la solution de lavage mise en oeuvre lors de l'étape (b) du procédé est choisie dans le groupe constitué par l'eau, l'eau distillée, l'eau déminéralisée, l'eau désionisée, un tampon phosphate salin (ou PBS), une solution saline (tel que NaCl), un tampon acétate (tel que AcONa), un tampon carbonate (tel que $NH_4HCO_3$ ou $NaHCO_3/Na_2CO_3$), une solution aqueuse comprenant un alcool et/ou un acide (i.e. la solution $T_1$ sans agent de coloration) telle que précédemment définie et leurs mélanges.

Avantageusement, le premier lavage lors de l'étape (b) du procédé selon l'invention est précédé du retrait de la solution $T_1$ contenant l'agent de coloration alors en contact avec la surface du support solide. Ce retrait peut être notamment effectué par tapotement, par absorption ou par aspiration.

Dans une forme de mise en oeuvre particulière, le dernier lavage est effectué avec de l'eau et notamment de l'eau distillée, déminéralisée et/ou désionisée (du type eau purifiée sur le système Milli-Q® de la société Millipore ci-après désignée par « eau Milli-Q »).

Après le dernier lavage de l'étape (b) du procédé selon l'invention, la surface du support solide peut être séchée avant la mise en contact avec la solution $T_2$ lors de l'étape (c).

L'analyse au spectrophotomètre du complexe agent de coloration/groupement protoné tel qu'amine après l'étape (b) du procédé de l'invention n'a pas été concluante. En effet, les complexes entre la molécule comprenant au moins un groupement protoné tel qu'une fonction amine et l'agent de coloration notamment le Bleu de Coomassie n'ont pu être détectés au spectrophotomètre car inférieurs au seuil de détection de la méthode. Rappelons qu'en solution et dans les conditions de Bradford, la limite de détection avoisine le μg/mL, soit, pour des puits de microplaque, 0,15 μg/puits. Or, après immobilisation sur support solide d'une interface de molécules comprenant au moins un groupement protoné tel qu'une fonction amine d'un poids moléculaire de 21500 Da, les inventeurs ont calculé que selon un modèle simplifié la monocouche sur le fond du puits n'excéderait pas 0,02 μg. Des lectures directes du complexe molécule-Bleu de Coomassie sur le support solide ne sont donc pas envisageables.

L'étape (c) du procédé selon l'invention dite « de décoloration » consiste à faire repasser en solution, grâce à une solution $T_2$, l'agent de coloration ayant interagi avec la (ou les) molécule(s) comprenant au moins un groupement protoné tel qu'une fonction amine présente(s) à la surface du support. Cette étape rend le procédé de l'invention « réversible » en permettant une réutilisation du support après sa caractérisation.

La solution $T_2$ est donc une solution apte à dissocier les éventuels complexes entre l'agent de coloration et la (ou les) molécule(s) comprenant au moins un groupement protoné tel qu'une fonction amine présente(s) à la surface du support. La composition de la solution $T_2$ a été optimisée de façon à décrocher la totalité de l'agent de coloration considéré fixé sur la surface lors de l'étape (a) de coloration.

Des tentatives pour dissocier les complexes entre l'agent de coloration et la molécule comprenant au moins une fonction amine, le support mis en oeuvre étant une plaque en polystyrène, ont été réalisées en utilisant des lavages intensifs avec soit de l'eau, des tampons acétate (AcONa), bicarbonate d'ammonium ($NH_4HCO_3$) et bicarbonate/carbonate de sodium ($NaHCO_3/Na_2CO_3$) à diverses molarités et à des pH allant jusqu'à 11,5, des solutions salines (NaCl (3M)), des solutions mixtes NaCl/tampon carbonate, soit des solutions alcooliques allant jusqu'à 50% d'alcool (tel que du méthanol ou de l'acétonitrile (ACN)). Ces tentatives appliquées à des supports en polystyrène sont restées sans succès et n'ont pas permis de décrocher l'agent de coloration du support solide.

Les mélanges en présence d'ACN ont été éliminés en raison de la faible résistance chimique à l'ACN de nombreux matériaux polymériques (notamment le polystyrène, matériau de base de nombreuses microplaques) susceptibles d'être utilisés pour le support solide ou sa surface.

Les inventeurs ont démontré qu'une solution mixte eau/ alcool appelée $T_2$ permet de dissocier les complexes entre l'agent de coloration et la molécule comprenant au moins un groupement protoné tel qu'une fonction amine. La

solution $T_2$ capable de décrocher l'agent de coloration de façon instantanée et quantitative est une solution aqueuse contenant un alcool et des ions carbonate.

Le pH de la solution $T_2$ est supérieur à 11 et, plus particulièrement, supérieur à 11, 25.

L'alcool contenu dans la solution $T_2$ est avantageusement choisi dans le groupe constitué par le méthanol, l'éthanol, le propanol, l'isopropanol et le butanol. La proportion d'alcool est comprise entre 40 et 60 % en volume par rapport au volume total de solution $T_2$.

La solution $T_2$ comprend, en plus d'un alcool des ions carbonate. Les ions carbonate contenus dans la solution $T_2$ présentent une molarité comprise entre 0,001 et 1 M, notamment comprise entre 0,005 et 0,5 M, en particulier comprise 0,01 et 0,1 M. Les ions carbonate de la solution $T_2$ mise en oeuvre dans le procédé selon l'invention se présentent avantageusement sous la forme de bicarbonate d'ammonium ($NH_4HCO_3$), de bicarbonate et carbonate de sodium ($NaHCO_3$/$Na_2CO_3$) ou de potassium ($K_2CO_3$), etc... ou de leurs mélanges.

L'étape (c) du procédé selon l'invention est effectuée à une température comprise entre 4 et 50°C et plus avantageusement à 20°C et ce sous agitation. La durée de l'étape (c) selon l'invention est supérieure à 1 seconde et inférieure à 5 min.

Le volume de solution $T_2$ devra être sélectionné de façon à recouvrir totalement la surface à caractériser. Ce volume à introduire lors de l'étape (c) ne devra cependant pas être trop important afin d'éviter une dilution trop importante de l'agent de coloration et permettre son dosage lors de l'étape (d). Une étape de concentration avantageusement par évaporation, peut être envisagée, suite à l'étape (c) et préalablement à l'étape (d), si d'importants volumes de décoloration ont été nécessaires.

L'étape (d) du procédé de l'invention consiste à détecter l'agent de coloration présent dans la solution $T_2$ récupérée après l'étape (c) i.e. la solution $T_2$ telle que précédemment définie comprenant en outre l'agent de coloration issu de la dissociation du complexe entre la molécule comprenant au moins un groupement protoné tel qu'une fonction amine et l'agent de coloration, lors de l'étape (c) du procédé.

Avant l'étape (d), la solution $T_2$ comprenant en outre l'agent de coloration récupéré lors de l'étape (c) a subi une acidification. Cette étape supplémentaire permet notamment de modifier le pH de la solution $T_2$ comprenant en outre l'agent de coloration récupéré lors de l'étape (c), afin de se placer dans des conditions optimales de pH en vue de la détection et de la quantification dudit agent de coloration.

La détection de l'agent de coloration lors de l'étape (d) du procédé selon l'invention consiste à mesurer la densité optique (ou absorbance) de la solution $T_2$ obtenue après l'étape (c) et acidifiée. La densité optique de cette solution est déterminée par un spectrophotomètre préalablement étalonné sur la longueur d'onde d'absorption de l'agent de coloration mis en oeuvre. Une telle détection est un travail de routine pour un homme du métier qui saura, en fonction de l'agent de coloration utilisé et du pH de la solution $T_2$, à quelle longueur d'onde d'absorption la détection de l'étape (d) doit être réalisée.

De plus, il est clair que, à la densité optique obtenue lors de l'étape (d) du procédé selon l'invention, on doit soustraire la densité optique obtenue, dans les mêmes conditions, en absence de la molécule comprenant au moins un groupement protoné tel qu'une fonction amine. Par « dans les mêmes conditions », on entend dans le cadre de la présente invention, même support, mêmes étapes (a) à (d) i.e. mêmes solutions $T_1$ et $T_2$, agent de coloration et quantité utilisés identiques, même(s) tampon(s) de lavage, mêmes températures, durée et condition pour chacune des étapes. La valeur obtenue après ladite soustraction est appelée « densité optique réelle » ou « absorbance réelle ». La densité optique mesurée en absence de molécule(s) comprenant au moins un groupement protoné tel qu'une fonction amine permet d'apprécier le bruit de fond de la mesure et les complexes éventuellement formés entre l'agent de coloration et le support solide, ses éventuels groupements fonctionnels, les éventuels bras espaceurs et/ou des molécules comprenant au moins un groupement protoné tel qu'une fonction amine éventuellement immobilisées sur le support solide et différentes des molécules comprenant au moins un groupement protoné tel qu'une fonction amine pour lesquelles le procédé de détection et éventuellement de quantification selon l'invention est mis en oeuvre.

Une densité optique obtenue lors de l'étape (d) du procédé selon l'invention en présence d'éventuelle(s) molécule(s) comprenant au moins un groupement protoné tel qu'une fonction amine supérieure à la densité optique obtenue, dans les mêmes conditions, en absence de molécule(s) comprenant au moins un groupement protoné tel qu'une fonction amine permet de conclure qu'au moins une molécule comprenant au moins un groupement protoné tel qu'une fonction amine est immobilisée à la surface du support solide.

La quantification de la (ou des) molécule(s) comprenant au moins un groupement protoné tel qu'une fonction amine à partir de la densité optique réelle obtenue lors de l'étape (d) du procédé selon l'invention peut être obtenue à partir de courbes d'étalonnage ou par calcul, comme présenté ci-après et notamment avec le Bleu de Coomassie utilisé comme agent de coloration.

Ainsi, l'étape (d) du procédé selon la présente invention peut comprendre les sous-étapes suivantes consistant à :

i) mesurer l'« absorbance réelle » (ou « densité optique réelle ») de l'agent de coloration dans la solution $T_2$ préa-

lablement acidifiée ;

ii) déduire de l'« absorbance réelle » mesurée à la sous-étape (i) la quantité et/ou la concentration d'agent de coloration présent dans la solution $T_2$, notamment à partir de courbes étalon de l'absorbance dudit agent de coloration en fonction de sa quantité et/ou de sa concentration ;

iii) déduire de la quantité et/ou de la concentration d'agent de coloration obtenue(s) à la sous-étape (ii) la quantité et/ou la concentration de groupements protonés tels que des fonctions amines et/ou de molécules comprenant au moins un groupement protoné tel qu'une fonction amine.

La sous-étape (iii) du procédé selon l'invention peut être mise en oeuvre en ayant préalablement déterminé le nombre de molécules d'agent de coloration susceptibles de se lier à un groupement protoné tel que précédemment défini et/ou à une molécule comprenant au moins un tel groupement protoné. Cette détermination préalable est notamment basée sur la loi de Lambert-Beer qui définit le changement d'absorbance ($\Delta A$) observé lorsque l'on ajoute des molécules comprenant un groupement protoné à une solution d'agent de coloration par l'équation (1) suivante

$$\Delta A = (a_{Bound} - a_{Free})\, l\, D_{Bound} \qquad (1)$$

dans laquelle

- $a_{Bound}$ et $a_{Free}$ sont les coefficients d'extinction molaires respectivement de l'agent de coloration lié et de l'agent de coloration libre ;
- $l$ est la longueur du trajet optique dans la solution traversée (à titre d'exemple, lorsque les expériences sont réalisées dans un puits de microplaque 96 puits, $l$ correspond à la hauteur de la solution dans le puits et est de l'ordre de 1,05 cm pour 300 $\mu$L de solution) et
- $D_{Bound}$ est la concentration d'agent de coloration lié.

Plus particulièrement, cette détermination préalable peut comprendre une ou plusieurs des étapes suivantes consistant à :

a) déterminer le coefficient d'extinction molaire $a_{Free}$ à partir de l'absorbance réelle obtenue pour des solutions contenant différentes quantités ou concentrations d'agent de coloration et de la loi de Lambert-Beer ;

β) déterminer le coefficient d'extinction molaire $a_{Bound}$ en présence d'un excès de molécules comprenant au moins un groupement protoné ;

Dans ce cas, tous les agents de coloration sont considérés liés ($D_{Bound}$) aux sites des molécules de façon à ce que $D_{Bound}$ égale la concentration initiale d'agent de coloration ($D_T$)

Ainsi, l'équation (1) de Lambert-Beer devient l'équation (2) suivante :

$$\Delta A = (a_{Bound} - a_{Free})\, l\, D_{Bound} = (\Delta a)\, l\, D_T \qquad (2)$$

dans laquelle $\Delta a$ est la différence des coefficients d'extinction molaires $a_{Bound} - a_{Free}$.

Lors de la détermination de $a_{Bound}$, le modèle mathématique de Atherton est utilisé.Les valeurs de $\Delta a$ et de $a_{Bound}$ peuvent être obtenues à partir de la représentation double-réciproque du changement d'absorbance réciproque ($1/\Delta A$) en fonction de la quantité ou de la concentration réciproque ($1/\Delta P_T$) de molécules comprenant au moins un groupement protoné à une concentration d'agent de coloration constante choisie ($D_T$).

ε) déterminer le nombre total de sites de liaison ($n_{Total}$) en présence d'un excés d'agent de coloration ;

En effet, un excès d'agent de coloration favorise la saturation de la liaison et un maximum des sites de liaison sont occupés par ledit agent de coloration i.e. $n = n_{Total}$. Aussi, $D_T \gg n_{Total}\, P_T$ avec $P_T$ représentant la concentration molaire en molécules comprenant au moins un groupement protoné ce qui entraîne que $D_{Bound} = n_{Total}\, P_T$ et que l'équation (1) de Lambert-Beer devient l'équation (3) ci-après :

$$\Delta A = (\Delta a)\, l\, n_{Total}\, P_T \qquad (3)$$

$n_{Total}$ peut donc être déterminé, en présence d'un excès d'agent de coloration, à partir de la courbe $\Delta A$ en fonction de $P_T$ et ce tant que cette courbe est linéaire. Ainsi, si et seulement si le nombre total théorique de sites de liaison a eté préalablement déterminé par une autre méthode analytique indépendante, le pourcentage de sites occupés par l'agent de coloration peut être calculé par molécule comprenant au moins un groupement protoné.

A titre d'exemple et lorsque l'agent de coloration mis en oeuvre est du Bleu de Coomassie, la solution $T_2$ est une solution composée à 50% de méthanol de qualité analytique et 50% d'une solution 0,1 M d'ions carbonate, avantageusement sous forme de bicarbonate/carbonate de sodium à un pH supérieur ou égal à 11,25. Dans ces conditions, le Bleu de Coomassie repasse en solution sous sa 4ème forme de couleur « violet-rose » (($\lambda_{max}$ autour de 530 nm, pH $\geq$ 11) peu évoquée jusqu'à présent dans la littérature. Dans la solution $T_2$, le Bleu de Coomassie libre présente une absorption maximale à 520 nm. Les mesures sont effectuées après ajout de 20 $\mu$L d'HCl (3 N) dans le tampon $T_2$, le pH est alors de 6,1. La forme libre de le Bleu de Coomassie vire au bleu et présente une absorption maximale à 610 nm stable. Il convient de remarquer que, compte tenu du spectre d'absorption du Bleu de Coomassie dans le tampon $T_2$ acidifié, l'absorption maximale s'étend entre 610 et 630 nm et que donc les mesures d'absorbance peuvent être effectuées à une quelconque longueur d'ondes incluse dans cette fourchette sans que le résultat en soit substantiellement affecté étant considéré que la gamme d'étalonnage est réalisée à la même longueur d'onde. Le nombre de molécules de Bleu de Coomassie détectées peut alors être corrélé à la densité de groupements protonés tels que des amines disponibles sur la surface à caractériser puisque les complexes formés entre le Bleu de Coomassie et la molécule comprenant au moins un groupement protoné tel qu'une fonction amine sont plus particulièrement formés entre le Bleu de Coomassie et ledit groupement protoné tel qu'une fonction amine.

Dans le but de quantifier le nombre de sites protonés tels que des amines présentes à la surface du support, les inventeurs ont réalisé des solutions étalons du Bleu de Coomassie dans la solution $T_2$ acidifiée (pH 6,1, $\lambda_{max}$ = 610 nm). La réponse est linéaire pour une concentration en Bleu jusqu'à 15 $\mu$g/mL et notamment comprise entre 0,5 et 15 $\mu$g/mL. La limite de quantification pour des puits de microplaques est alors de 39,5 ng/mL (coefficient de variation (CV) < 2 % ; n=64). Ceci représente un minimum de $10^{13}$ molécules de Bleu de Coomassie quantifiables par $cm^2$. Les valeurs reportées sur la Figure 2A sont la moyenne de 6 mesures réalisés dans la même journée. Les écarts-types sur la mesure sont inférieurs à 0,02 unités d'absorbance. Ces gammes ont été répétées à plusieurs reprises à des jours différents et avec n = 3 manipulateurs différents (fidélité intermédiaire, pente = 0,0802, CV < 1 %). Les tests aux résidus réduits montrant une répartition très aléatoire des valeurs sont reportés sur la Figure 2B.

Dans le cadre de cette approche, les inventeurs ont cherché à définir le nombre maximal de molécules de Bleu de Coomassie susceptibles d'interagir par molécule présentant au moins un groupement protoné tel qu'une fonction amine lors de la formation de complexes entre cette molécule et le Bleu de Coomassie. La finalité est de définir un rapport nombre de molécules de Bleu de Coomassie par nombre de groupements protonés tels que des amines.

Selon l'équipe de recherche de Splittgerber [Chial, 1993][Congdon, 1993], en milieu homogène, le nombre moyen de molécules de Bleu de Coomassie liées par molécule de protéine est donné par l'équation (Eq.3).

Dans le cas de la présente invention, la solution $T_1$ présente un nombre de molécules de Bleu de Coomassie en très grand excès. La valeur de **$n_{total}$** représentant le nombre total de molécules du Bleu de Coomassie ayant interagi avec les groupements protonés tels que les amines de surface du support solide est déterminée lors de la phase de décoloration de la présente invention c'est-à-dire lors du dosage du Bleu de Coomassie après décrochage dans la solution $T_2$. Cette valeur est donc directement proportionnelle au nombre de groupements protonés tels que des amines du support ayant réagi lors de l'étape (a) du procédé.

En suivant un modèle mathématique à deux niveaux de concentration en bleu de Coomassie décrit par Atherton [Atherton, 1996], les inventeurs ont démontré, que dans les conditions de saturation, le pourcentage de sites occupés par le bleu de Coomassie était de 93% sur la sérum albumine bovine, de 93,5% sur la poly(lysine) linéaire, de 91,7% sur un dendrimère de la lysine de génération 2, de 92,7% pour la génération 3, et 89,9% pour la génération 4 et de 96,9% pour un dendrimère de type PAMAM de génération 5, soit qu'il y a au plus **une molécule de bleu par groupement protoné tel que $NH_2$.**

Sur la base de l'enseignement ci-dessus, l'homme du métier peut mettre en oeuvre la présente invention, sans effort inventif, en utilisant un agent de coloration autre que le Bleu de Coomassie et notamment un agent de coloration capable de former des complexes avec une molécule comprenant au moins un groupement protoné, tel qu'une fonction amine, sans que ce dernier soit directement impliqué dans le complexe formé.

Les applications de la présente invention colorimétrique sont multiples et variées.

Ainsi, la présente invention concerne l'utilisation du procédé tel que précédemment défini pour comparer différentes stratégies (ou protocoles) d'immobilisation de molécules comprenant au moins un groupement protoné tel qu'une fonction amine sur des supports solides et susceptibles de générer des supports coatés, des puces à peptides, des puces à protéines, des puces à anticorps ou des puces à cellules.

La présente invention concerne également l'utilisation du procédé tel que précédemment défini pour vérifier si les supports coatés, les puces à peptides, les puces à protéines, les puces à anticorps ou les puces à cellules répondent à tous les critères de qualité analytique. En effet, la présente invention permet de vérifier tous les critères de qualités analytiques que sont :

- la **stabilité** du support recouvert d'une interface comprenant une ou des molécule(s) comprenant au moins une fonction amine aux cycles de lavage et/ou de coloration/décoloration de la présente invention,

- la **robustesse** *(répétabilité et reproductibilité)* des greffages de ladite interface dans la fabrication de supports coatés, de puces à peptides, de puces à protéines, de puces à anticorps ou de puces à cellules (homogénéité intra et inter expériences),
- la **sensibilité** par comparaison aux supports coatés, puces à peptides, puces à protéines, puces à anticorps ou puces à cellules commerciaux, etc..

La présente invention concerne aussi l'utilisation du procédé tel que précédemment défini pour détecter et quantifier des molécules comprenant au moins un groupement protoné tel qu'une fonction amine et plus particulièrement des protéines, des anticorps ou leurs fragments présents dans un liquide d'intérêt. Par « liquide d'intérêt », on entend dans le cadre de la présente invention un milieu de culture cellulaire, un fluide biologique préalablement isolé ou extrait du corps humain ou animal (du type sang, lymphe, urine, salive, sperme, etc...) . La mise en oeuvre de cette application particulière comprend les étapes successives suivantes :

- préparation d'un support solide présentant à sa surface au moins un antigène ou au moins un anticorps spécifique de la protéine, de l'anticorps ou de leurs fragments recherchés ;
- mise en contact du support ainsi préparé avec le liquide d'intérêt ;
- étape (a) du procédé selon l'invention telle que précédemment définie ;
- étape (b) du procédé selon l'invention telle que précédemment définie ;
- étape (c) du procédé selon l'invention telle que précédemment définie ;
- étape (d) du procédé selon l'invention telle que précédemment définie.

Il est clair que, lors de la mise en oeuvre de l'étape (d) et ce en vue de détecter ou de quantifier au moins une protéine, au moins un anticorps ou au moins un de leurs fragments présents dans un liquide d'intérêt, la densité optique réelle telle que précédemment définie est obtenue en soustrayant, à la densité optique obtenue à l'étape (d), la densité optique obtenue après la mise en oeuvre du procédé selon l'invention sur le support solide présentant à sa surface au moins un antigène ou au moins un anticorps spécifique de la protéine, de l'anticorps ou de leurs fragments recherchés.

La description propose également la solution $T_1$ telle que précédemment définie contenant un agent de coloration tel que précédemment défini en une quantité supérieure ou égale à 0,05 % en masse par rapport au volume de la solution $T_1$.

La description propose également la solution $T_2$ telle que précédemment définie i.e. une solution comprenant un alcool et des ions carbonate.

La présente invention concerne un kit d'éléments tel qu'un kit de dosage pour la mise en oeuvre du procédé selon l'invention contenant :

- dans un premier compartiment, la solution $T_1$ telle que précédemment définie ;
- dans un second compartiment, la solution $T_2$ telle que précédemment définie ;
- et, éventuellement, une (ou plusieurs) solution(s) de lavage telle(s) que précédemment définie(s).

Avantageusement, le kit d'éléments selon l'invention comprend

- dans un premier compartiment, la solution $T_1$ telle que précédemment définie ;
- dans un deuxième compartiment, la solution $T_2$ telle que précédemment définie ;
- dans un troisième compartiment, une solution de lavage telle que précédemment définie ;
- dans un quatrième compartiment, une solution pour acidifier la solution $T_2$.

Par « compartiment », on entend dans le cadre de la présente invention un récipient unique tel qu'un flacon qu'un ensemble de récipients, identiques ou différents, contenant la même solution tels que des ampoules.

Un exemple particulier de kit d'éléments selon l'invention et notamment utilisable pour réaliser 320 mesures, comprend :

- dans un premier compartiment, 100 ml de Bleu de Coomassie à 500 mg.mL$^{-1}$ dans un tampon acide acétique/ méthanol/eau ultrapure (v :v :v ; 5 :10 :85) (Réactif A) ;
- dans un deuxième compartiment, 100 ml de tampon carbonate 0,25 M, pH 11,25 à 50% dans méthanol (Réactif B) ;
- dans un troisième compartiment, 750 mL d'une solution de lavage comprenant acide acétique/ méthanol/eau ultrapure (v :v :v ; 5 :10 :85) (Réactif C) ;
- dans un quatrième compartiment, 8 ampoules de 1 mL d'acide chlorhydrique (3N),

ledit kit pouvant être accompagné d'une notice explicative précise du protocole à suivre du principe de la mesure ainsi que des conseils en cas de problèmes lors de la mise en oeuvre du kit ou du procédé du type « troubleshooting ».

La description propose enfin l'utilisation de la solution $T_1$ telle que précédemment définie, de la solution $T_2$ telle que précédemment définie, de la forme de bleu de Coomassie « violet-rose pâle » présentant une absorption maximale à 520 nm et/ou de la forme de bleu de Coomassie « bleu turquoise » présentant une absorption maximale à 610 nm pour détecter et éventuellement quantifier, de façon directe, au moins une molécule comprenant au moins un groupement protoné tel qu'une fonction amine immobilisé à la surface d'un support solide.

D'autres caractéristiques et avantages de la présente invention apparaîtront encore à la lecture des exemples ci-après donnés à titre illustratif et non limitatif.

## BRÈVE DESCRIPTION DES DESSINS

La Figure 1 est une schématisation des étapes principales du procédé selon l'invention.

La Figure 2 présente la détermination du domaine de linéarité du Bleu de Coomassie dans la solution $T_2$/HCl (Figure 2A) et le test aux résidus réduits (Figure 2B).

La Figure 3 présente la valeur d'absorbance du Bleu de Coomassie dans la solution $T_2$/HCl, mesurée à 620 nm, en fonction du pH de ladite solution $T_2$/HCl.

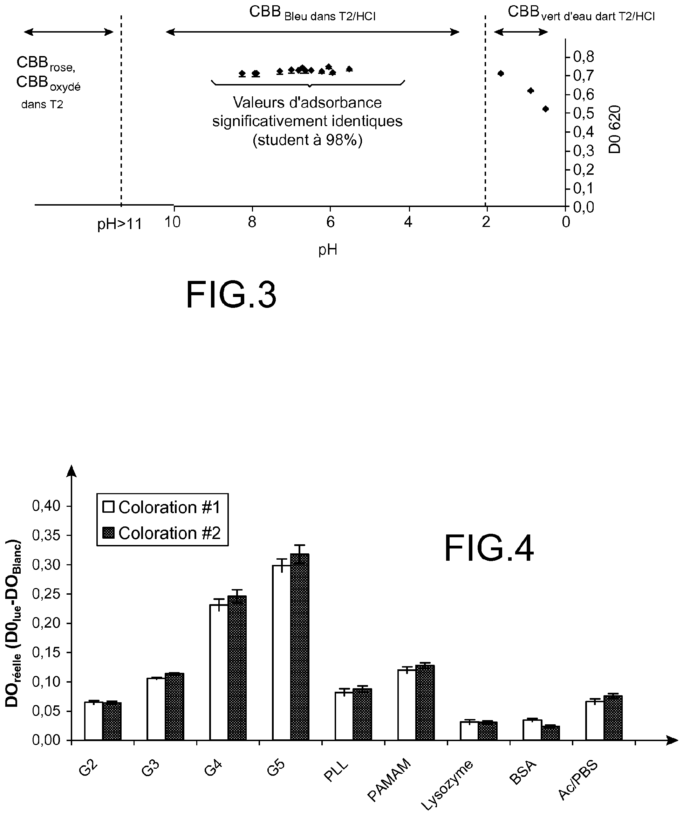

La Figure 4 présente l'absorbance réelle du Bleu de Coomassie dans la solution $T_2$/HCl mesurée après un seul cycle de coloration/décoloration (coloration 1) ou après deux cycles de coloration/décoloration (coloration 2) et ce pour différentes molécules immobilisées sur un support solide en polystyrène qui sont des dendrimères greffés de lysine (demande internationale WO 2006/114528) de génération 2 (G2), de génération 3 (G3), de génération 4 (G4) et de génération 5 (G5) ; des dendrimères polyamidoamine (PAMAM) ; des protéines naturelles (sérum albumine bovine (BSA) et lysosyme) ; des protéines synthétiques (polylysine linéaire (PLL)) et des anticorps monoclonaux (Ac/PBS).

## EXPOSÉ DÉTAILLÉ DE MODES DE RÉALISATION PARTICULIERS

## EXEMPLE 1: CARACTERISATION D'UN SUPPORT COMMERCIAL PRESENTANT DES ALKYLAMINES ($NH_2$).

### 1. Matériel.

**Format :** Microplaques de 96 puits ;
**Matériau :** polystyrène ;
**Recouvrement « Coating » :** alkylamine (amine primaire terminale), Amine™ Corning (#2388).

### 2. Protocole de caractérisation.

Etape préliminaire - Conditionnement : sont introduits par puits 300 $\mu$L de solution $T_1$ (85% d'eau milliQ, 10% de méthanol de qualité analytique, 5% acide acétique de qualité analytique). Cette solution est laissée en contact dans le puits, sous agitation, pendant 15 min, à température ambiante.

Etape (a) du procédé de l'invention : sont introduits par puits 100 $\mu$L de Bleu de Coomassie (G250) à 500 $\mu$g/mL dans la solution $T_1$. Cette solution est laissée en contact dans le puits, sous agitation, pendant 15 min, à température ambiante, à l'obscurité.

Etape (b) du procédé de l'invention : les puits sont vidés, rincés trois fois avec 300 $\mu$L de $T_1$, puis sont ajoutés par puits 300 $\mu$L de $T_1$. Cette solution est laissée en contact dans le puits, sous agitation, pendant 15 min, à température ambiante.

Etape (c) du procédé de l'invention : Les puits sont vidés. Une microplaque native Maxisorp™ (Nunc) est préparée avec 20 $\mu$L d'acide chlorhydrique 3N par puits. La plaque ayant précédemment interagi avec le Bleu de Coomassie est « décolorée »avec 300 $\mu$L de tampon $T_2$ (50% de tampon carbonate 0,1 M pH 11,25 contenant 50% de méthanol de qualité analytique), et 270 $\mu$L sont prélevés en un temps inférieur à 5 min pour les déposer sur la plaque Maxisorp™ précédemment préparée.

Etape (d) du procédé selon l'invention : L'absorbance des solutions récupérées acidifiées est alors mesurée à 620 nm. Dans les exemples qui suivent les absorbances réelles sont considérées. Les absorbances réelles sont le résultat de la différence des absorbances lues avec celles des bruits de fond obtenues dans les mêmes conditions (par exemple même solution tampon mais sans bleu de Coomassie...).

### 3. Résultats.

Tableau 1

|  | NH$_2$/cm$^2$ donné par le fabricant | NH$_2$/cm$^2$ mesuré par l'invention |
|---|---|---|
| Amine™ Corning | 2 10$^{13}$ | 0,27 10$^{13}$ $\pm$ 2% (n=48) |

Les résultats présentés au Tableau 1 ci-dessus montrent que l'invention permet de déterminer le nombre d'amine de surface de façon sensible et répétable.

Le temps total de caractérisation a été de 55 minutes pour 48 puits, il est au minimum de 50 min pour un puits. Aucun matériel lourd n'est nécessaire pour réaliser ce dosage qui est aisément réalisé en 4 étapes. La présente invention est donc simple de mise en oeuvre, rapide et répétable.

**EXEMPLE 2** : **Linéarité, limite de quantification (LOQ) et robustesse de la méthode décrite par la présente invention.**

### 1. Etude de la linéarité.

La linéarité du procédé selon l'invention a déjà été discuté (voir Figure 2).

### 2. Coefficient de variation à la LOQ.

La LOQ a été estimée par mesure d'un volume de 290 $\mu$L d'une solution T$_2$ + HCl (comme défini dans l'exemple 1) correspondant à un blanc avec LOQ = DO$_{blanc}$+10*$\sigma_{DOblanc}$ sur 16 mesures.

Une fois cette LOQ estimée, 4 solutions de Bleu de Coomassie ont été préparées à la LOQ dans la solution T$_2$ + HCl par trois manipulateurs différents à partir de Bleu de Coomassie commercial (G-250). Ces 4 solutions ont été mesurées 16 fois (290 $\mu$L/puits) à 620 nm.

**Résultats**

La LOQ est de 39,5 ng de Bleu de Coomassie/mL, cette LOQ pouvant être améliorée en concentrant la solution par évaporation avant d'effectuer la mesure.

Le coefficient de variation à la LOQ est de 1,64% (n=64, reproductibilité).

### 3. Fidélité de la solution mère de Bleu de Coomassie dans T$_1$.

7 solutions mères de Bleu de Coomassie dans T$_1$ (500 $\mu$g/mL) ont été préparées par 4 manipulateurs différents, sur 10 jours. Les DO de ces 7 solutions ont été mesurées après dilution au 1/50 dans T$_1$ (270 $\mu$L, à 620 nm.) Ces solutions ont ensuite été utilisées pour « colorer » des plaques amine™ de chez Corning comme décrit dans l'exemple 1.

**Résultats**

La DO des solutions mères de Bleu de Coomassie dans T$_1$ est comprise entre 0,39-0,5 unité d'absorbance.

La coloration des plaques avec les deux solutions extrêmes préparées (DO 0,39 et DO 0,5) a conduit à une DO respectivement de 0,168 $\pm$<2% et 0,167 $\pm$<2% (n=8 dans les deux cas). Ces résultats montrent que la préparation des solutions mères de Bleu de Coomassie conduit à des colorations reproductibles.

### 4. Robustesse de la mesure en fonction du pH du Tampon T$_2$ + HCl.

L'influence de la molarité de l'HCl (1-6N) et du volume d'HCl (5,4%-12%) dans T$_2$ correspondant à des pH compris entre 0,1-11,2 a été testée.

Les résultats montrent (Figure 3) qu'une variation de 5,7% (correspondant à $\pm$ 1,1 $\mu$L d'HCl 3N) sur le volume d'HCl 3N ajouté à T$_2$ n'induit aucune différence significative (test de Student à 98%) sur la DO mesurée.

L'ensemble de ces résultats montre que la méthode décrite dans la présente invention est sensible, fidèle et robuste.

## 5. Robustesse de la mesure en fonction du co-solvant de $T_2$+HCl.

L'influence du co-solvant organique (méthanol, éthanol) a été testé sur des microplaques de 96 puits en polystyrène coaté avec des amines (Amine™ Corning # 2388) qui après conditionnement, coloration et lavages selon le protocole décrit exemple 1, ont été décolorées soit avec un tampon $T_2$ tel que décrit exemple 7, soit avec un tampon $T_2$ dans lequel le méthanol a été remplacé par l'éthanol dans les mêmes proportions.

**Résultats**

Les densités optiques mesurées à 620 nm sont de :

- $T_2$/méthanol : 0,151 $\pm$ 0,0087 (n=6),
- $T_2$/éthanol : 0,147 $\pm$ 0, 0064 (n=6).

Les résultats obtenus avec l'éthanol et le méthanol sont statistiquement identiques (Student à 95%). Il est donc possible de remplacer le méthanol par l'éthanol.

**EXEMPLE 3** : **UTILISATION DE LA PRESENTE INVENTION POUR CARACTERISER DES AMINES DE SURFACE SUR DES SUPPORTS DE FORMAT ET DE MATERIAU DIFFERENTS.**

## 1. Tube à hémolyse en verre.

**Format** : Tube à hémolyse
**Matériaux :** Verre
**Coating :** Ces tubes ont été activés selon la méthode décrite par Arkas *et al.* 2005, Metwalli *et al.* 2006 et Pathak *et al.* 2004. Après activation, les tubes ont été greffés avec des Dendrimère Greffés de la Lysine (demande internationale WO 2006/114528) de génération 3 (DGL-G3) en utilisant le protocole décrit par Arkas *et al.* 2005, Metwalli *et al.* 2006 et Pathak *et al.* 2004. Après le greffage, les supports sont lavés comme précédemment décrit. Ensuite, le greffage des DGL-G3 a été caractérisé selon le protocole de l'invention décrit exemple 1 avec un volume de coloration (étape a) et de décoloration (étape b) de 3,5 mL.
**Résultats** : $DO_{réelle}$ 0, 056 $\pm$ 0,08 (n=7), soit 3,56 $10^{14}$ $NH_2/cm^2$.

## 2. Microplaques en COC, polystyrène et polypropylène.

**Format :** microplaques de 24 ou 96 puits
**Matériaux :** COC, polystyrène et polypropylène
**Coating :** voir ci-dessous

- Plaques COC-24 puits de chez Greiner (fait à façon par le fournisseur) fonctionnalisés en surface par des époxydes, greffage de DGL-G3 (200 $\mu$g/mL en tampon carbonate 0,1 M pH 9,5) à température ambiante 16 h puis lavés comme précédemment décrit ;
- Polystyrène de 96 puits, Corning : voir exemple 1 ;
- Polypropylène de 96 puits de chez Greiner (#655201) activé par plasma d'argon suivi de la polymérisation du couple N-acryloyl-N-Morpholine et N-acryloyl-N-Succinimide (NAM/NAS) dans le dioxane, lavage à l'eau et greffage avec du DGL-G3 à 1 mg/mL dans le tampon PBS 0,2 M, pH 7,4. Caractérisation selon le protocole décrit exemple 1, après lavage de l'excès de DGL-G3 comme décrit pour le COC.

**Résultats :**

Tableau 2

| Support et greffage | x $10^{14}$ NH$_2$ /cm$^2$ | |
|---|---|---|
| COC-DGL-G$_3$ | 1,47 $\pm$ 17% (n=24) | |
| Polypropylène-NAM/NAS-DGL-G$_3$ | 4,1 $\pm$ 4,6% (n=47) | Coloration 1 |
| | 4,0 $\pm$ 4,6% (n=47) | Coloration 2 |
| Avec n= nombre de mesures | | |

Les résultats présentés dans le Tableau 2 montrent que les coefficients de variation des greffages sur les supports COC sont importants et fortement supérieurs à ceux obtenus sur le polypropylène. Ceci révèle un problème d'homogénéité des greffages sur la plaque COC alors que la méthode d'activation-greffage utilisée sur le polypropylène est homogène sur l'ensemble de la microplaque. Ainsi, la méthode décrite par la présente invention permet d'étudier l'homogénéité des greffages sur une surface.

La méthode décrite dans la présente invention conduit à des résultats de coloration identiques d'un cycle à l'autre (Tableau 2). Ainsi, les amines « colorées » au premier cycle (coloration 1) puis « décolorées » avant la seconde « coloration », ont toutes été libérées, et ont pu être « colorées » une seconde fois (coloration 2) pour conduire à un résultat significativement identique à la première mesure. Ceci démontre que la présente invention 1/ permet de vérifier la stabilité des greffages mais aussi 2/ est parfaitement réversible.

Enfin, la présente invention qui conduit à l'évaluation de la densité des fonctions amines par unité de surface (NH$_2$/cm$^2$) permet de comparer entre eux des supports de matériaux, de format et de greffage différents.

**3. Feutres et pastilles en polypropylène.**

**Format** : Feutres de 5 mg ou pastilles de 6 mm de diamètre

**Matériaux** : polypropylène

**Coating** : identique à la méthode d'activation décrite ci-dessus pour les microplaques 96 puits en polypropylène, greffage avec du DGL-G$_3$. La caractérisation est réalisée selon le protocole décrit exemple 1.

**Résultats :**

Tableau 3

| Support de greffage | x $10^{14}$ NH$_2$/mg |
|---|---|
| Feutres (5mg) | 1,36 $\pm$ 0,08 (CV 5,8%) |
| Pastilles (3.7 mg - 6 mm Ø) | 1,059 $\pm$ 0,025 (CV 2,4%) |
| avec CV : coefficient de variation | |

L'ensemble des résultats présentés dans l'exemple 3 montre que la méthode décrite dans la présente invention pour la détection et la quantification des amines en surface d'un support solide est quantitative, réversible, adaptée à de nombreux matériaux, formats et coating tels que précédemment décrits. La méthode décrite permet de vérifier l'homogénéité et la stabilité des greffages.

**EXEMPLE 4** : UTILISATION DE LA PRESENTE INVENTION POUR CARACTERISER L'INSTABILITE DES GREFFAGES DE PROTEINES ADSORBEES SUR DES SUPPORTS SOLIDES.

**Format** : Microplaques de 24 et 96 puits

**Matériaux** : polystyrène (Greiner et Nunc)

**Coating** :

- Greiner : Cellcoated© poly-L-lysine (ref 655930) 96 puits et format 24 puits fait sur demande par le fournisseur selon le protocole "Cellcoated© poly-L-lysine" ;

- Nunc : Microwell poly-D-lysine référence : 152039

**Résultats :**

Tableau 4

| Support de greffage | Coloration 1 x $10^{14}$ NH$_2$/cm$^2$ | Coloration 2 x $10^{14}$ NH$_2$/cm$^2$ |
|---|---|---|
| Greiner-PLL 96 puits | 0,514 (CV 11%, n=96) | BF |
| Greiner -PLL 24 puits | 1,471 (CV 14%, n=24) | 0,927 (CV14%, n=24) |
| NUNC - PDL 96 puits | 0,232 (CV 27%, n=96) | LOD<DO<LOQ |
| avec PLL= poly-L-Lysine, PDL= Poly-D-Lysine, BF=bruit de fond, CV= coefficient de variation, LOD= limite de détection, LOQ= limite de quantification | | |

Les résultats du Tableau 4 montrent que la méthode décrite dans le présent brevet permet de mettre en évidence l'inhomogénéité des greffages (ici par adsorption) ainsi que l'instabilité du greffage lorsque les protéines sont adsorbées et non pas greffées de façon covalente sur le support.

## EXEMPLE 5: REPRODUCTIBILITE DES GREFFAGES INDIRECTS UTILISANT DU GLUTARALDEHYDE

L'immobilisation de l'interface dendrimérique DGL (génération G3) a été réalisée via un lien homobifonctionnel (le glutaraldéhyde) sur un support fonctionnalisé en surface par des amines (NHR, Covalink™). Le protocole est couramment utilisé au laboratoire pour la fixation de l'histamine [Claeys-Bruno, 2006]. Le volume de solution est de 200 $\mu$L/puits. Les résultats obtenus sont présentés dans le tableau 4 ci-après.

Tableau 5

| Concentration introduite | Concentration en bleu ($\mu$g/mL) |
|---|---|
| Sans DGL « support commercial NHR» | 0,16 $\pm$ 0,01 |
| 100 $\mu$g/puits | 5,0 $\pm$ 0,3 |
| 200 $\mu$g/puits | 6,0 $\pm$ 1,0 |
| 400 $\mu$g/puits | 5,1 $\pm$ 0,7 |
| 1000 $\mu$g/puits | 5,3 $\pm$ 0,8 |

Ce travail a permis, dans un premier temps, de mettre au point les conditions optimales de lavage pour éliminer au maximum les agrégats possibles de DGL et les adsorptions non spécifiques sur le support. Les valeurs présentées dans le tableau 5 sont obtenues après 3 bains de lavage en tampon carbonate/50%MeOH. De même, après 6 cycles de coloration/décoloration sur ces supports coatés, la valeur du bleu de Coomassie dans T$_2$ ne varie pas par puits, les valeurs de bleu étant identiques au seuil des 95% pour un même puits après des cycles de coloration/décoloration.

Dans un second temps, grâce à la présente invention, il a été démontré qu'une concentration inférieure à 100 $\mu$g de DGL (G3) suffit à la formation d'un support coaté dans le cas de la stratégie utilisant le glutaraldéhyde comme agent bifonctionnel.

Or, la présente stratégie a également permis de mettre en évidence qu'il existe une grande variabilité sur les valeurs obtenues sur des greffages réalisés sur deux journées différentes et parfois même d'un puits à l'autre avec cette stratégie de greffage utilisant le glutaraldéhyde comme agent bifonctionnel. Il a donc pu être vérifié dans ce cas particulier qu'une stratégie d'immobilisation utilisant le lien glutaraldéhyde ne conduit pas à une homogénéité de greffage. Les supports ainsi coatés ne répondent donc pas au critère de reproductibilité.

Quelle que soit la concentration en DGL introduite, les supports coatés conduisent à des concentrations en bleu de Coomassie plus de 30 fois supérieures à celles du support commercial. En outre, des greffages de DGL par adsorptions sur le support montrent qu'après des lavages dans des bains de tampon carbonate/MeOH, les valeurs de bleu obtenues dans $T_2$ ne sont pas significativement différentes de celles obtenues sur le support NHR. Ceci suggère que :

1/ il y a bien élimination des adsorptions non spécifiques
2/ que les valeurs lues sont corrélées uniquement aux DGL greffés de façon covalente au support
3/ la stratégie d'immobilisation de l'interface DGL *via* le glutaraldéhyde permet néanmoins d'obtenir des densités d'amines de surface beaucoup plus importantes que la précédente stratégie et par rapport au support commercial puisqu'il y a environ 8 x $10^{14}$ molécules de bleu par puits après l'étape de décoloration (gain en sensibilité).

**EXEMPLE 6** : **UTILISATION DE LA PRESENTE INVENTION POUR CARACTERISER DIFFERENTES PROTEINES IMMOBILISEES SUR UN SUPPORT SOLIDE (DENDRIMERES SYNTHETIQUES, PROTEINES SYNTHETIQUES ET NATURELLES, ANTICORPS).**

**Format** : Microplaques de 96 puits
**Matériaux** : polystyrène
**Coating** : plaques fonctionnalisées en surface par des époxydes (Nunc, immobilizer NH$_2$™). Les protéines sont greffées à 200 $\mu$g/mL dans le tampon carbonate 0,1 M pH 9 excepté pour les anticorps qui sont greffés dans le tampon PBS 0,2 M, pH 7,4 comme décrit pour les plaques de polypropylène dans l'exemple 9.

Tableau 6

| Protéines | Masse moléculaire Dalton |
|---|---|
| Dendrimères Greffés de la Lysine (WO 2006/114528) | |
| G2 | 8 350 |
| G3 | 21 500 |
| G4 | 64 000 |
| G5 | 169 000 |
| Polylysine linéaire -Sigma | 15-30 000 |
| PAMAM G5 (Sigma) | 28 825 |
| Lysozyme (Fluka) | 16 000 |
| Sérum albumine bovine (BSA) | 69 000 |
| Anticorps monoclonaux (Pierce # 0031242) | 150 000 |

**RESULTATS** : La figure 4 montre que la méthode décrite permet aussi bien de caractériser des amines de surface issues de protéines naturelles que de synthèse et ce de manière répétable. Ainsi la méthode peut être utilisée sur des protéines de masse et d'origine variable (naturelle, synthèse) ainsi que sur des amines de synthèse (voir exemple 1).

**Références bibliographiques**

Arkas M., Tsiourvas D ., Paleos C.M. (2005). "Organosilicon Dendritic Networks in Porous Ceramics for Water Purification." Chemistry of Material, 17: 3439-44.
Atherton, B. A., E.L. Cunningham and A.G. Splittgerber (1996). "A mathematical model for the description of the coomassie brilliant blue protein assay." Analytical Biochemistry 233: 160-168.
Bradford, M. M. (1976). "A rapid and sensitive method for the quantitation of microgram quantifies of protein utilizing the principle of protein-dye binding." Analytical Biochemistry 72: 248-254.
Chial, H. J., H. B. Thompson, et al. (1993). "A spectral study of the charge forms of Coomassie blue G." Analytical Biochemistry 209(2): 258-266.
Chial, H. J. and A. G. Splittgerber (1993). "A comparison of the binding of Coomassie brilliant blue to proteins at low and neutral pH." Analytical Biochemistry 213(2): 362-369.
Congdon, R. W., G. W. Muth, et al. (1993). "The binding interaction of Coomassie blue with proteins." Analytical Biochemistry 213(2): 407-413.

Claeys-Bruno, M., O. Vandenabeele-Trambouze, et al. (2006). "Methodological approaches for histamine quantification using derivatization by chloroethylnitrosourea and ELISA measurement. Part I. Optimization of derivated histamine detection with coated-plates using optimal design and Part II: Optimisation of the derivatization step." Chemometrics and Intelligent Laboratory Systems 80(2): 176-197.

Metwalli E., Haines D. Becker O., Conzone S., Pantano C.G. (2006) "Surface characterizations of mono-, di-, and tri-aminosilane treated glass substrates." Journal of Colloid and Interface Science, 298: 8252-31.

Pathak S. Singh A.K., McElhanon J.R., Dentinger P.M. (2004) "Dendrimer-activated surfaces for high density and high activity protein chip applications" Langmuir, 20: 6075-79.

Brevet US 6,696,304 (Luminex corporation) publié le 24 février 2004.

Brevet US 6,555,382 (Wondrak E.M.) publié le 29 novembre 2001.

Demande internationale WO 2006/114528 (CNRS & UNIVERSITE MONTPELLIER II) publiée le 2 novembre 2006.

## Revendications

1.  Procédé de détection et de quantification d'au moins une molécule comprenant au moins un groupement protoné immobilisée à la surface d'un support solide, ledit procédé comprend les étapes successives suivantes :

    a) mise en contact de ladite surface du support solide sur laquelle est immobilisée au moins une molécule comprenant au moins un groupement protoné avec une solution $T_1$ contenant du bleu de Coomassie G250 comme agent de coloration,
    ledit groupement protoné étant choisi dans le groupe constitué par une fonction amine, une fonction imine, une fonction guanidino et un groupement hétéroaryle,
    b) élimination dudit agent de coloration n'ayant pas réagi avec ladite surface lors de l'étape (a),
    c) mise en contact de ladite surface avec une solution $T_2$ apte à dissocier l'éventuel (ou les éventuels) complexe(s) formé(s) entre ledit agent de coloration et ladite molécule comprenant au moins un groupement protoné, ladite solution $T_2$ étant une solution aqueuse contenant un alcool dans une proportion comprise entre 40% et 60% en volume par rapport au volume total de la solution $T_2$ et des ions carbonates,
    le pH de ladite solution $T_2$ étant supérieur à 11,
    d) acidification de ladite solution $T_2$ et détection dudit agent de coloration éventuellement présent dans la solution $T_2$ acidifiée de façon à détecter et quantifier ladite molécule comprenant au moins un groupement protoné,

    ladite détection de l'agent de coloration lors de l'étape (d) consistant à mesurer la densité optique de la solution $T_2$ acidifiée obtenue après l'étape (c),
    ladite détection et ladite quantification de ladite molécule comprenant au moins un groupement protoné étant obtenues à partir de la densité optique réelle correspondant à la densité optique de la solution $T_2$ acidifiée obtenue après l'étape (c) à laquelle a été soustraite la densité optique obtenue, dans les mêmes conditions mais en absence de ladite au moins une molécule comprenant au moins un groupement protoné.

2.  Procédé selon la revendication 1, **caractérisé en ce que** ladite molécule comprenant au moins un groupement protoné est choisie dans le groupe constitué par les peptides, les polypeptides, les protéines, les protéines présentant des groupes prosthétiques, les anticorps, les acides nucléiques transaminés, les amines biogènes, les dendrons et les dendrimères, leurs fragments et leurs dérivés.

3.  Procédé selon la revendications 1 ou 2, **caractérisé en ce que** ledit support solide ou du moins ladite surface dudit support solide où ladite molécule comprenant au moins un groupement protoné est immobilisée, est un support solide ou une surface inorganique.

4.  Procédé selon la revendications 1 ou 2, **caractérisé en ce que** ledit support solide ou du moins ladite surface dudit support solide où ladite molécule comprenant au moins un groupement protoné est immobilisée est en un matériau organique.

5.  Procédé selon l'une quelconque des revendications précédentes, **caractérisé en ce que** ledit support solide présente une surface portant des groupements fonctionnels grâce auxquels ladite molécule comprenant au moins un groupement protoné est capable de s'immobiliser.

6.  Procédé selon l'une quelconque des revendications précédentes, **caractérisé en ce que** ladite molécule comprenant au moins un groupement protoné est immobilisée de façon indirecte à la surface du support solide.

7. Procédé selon l'une quelconque des revendications précédentes, **caractérisé en ce que** ladite solution $T_1$ est une solution aqueuse comprenant un alcool et/ou un acide, le pH de ladite solution $T_1$ étant, de préférence, supérieur à 1.

8. Procédé selon la revendication 7, **caractérisé en ce que** ledit alcool est choisi dans le groupe constitué par le méthanol, l'éthanol, le propanol, l'isopropanol, le butanol et leurs mélanges, la proportion d'alcool étant, de préférence, comprise entre 1 et 25 % en volume par rapport au volume total de solution $T_1$.

9. Procédé selon la revendication 7 ou 8, **caractérisé en ce que** ledit acide est choisi dans le groupe constitué par l'acide acétique, l'acide trichloroacétique et leurs mélanges, la proportion d'acide étant, de préférence, comprise entre 0,5 et 20 % en volume par rapport au volume total de solution $T_1$.

10. Procédé selon l'une quelconque des revendications précédentes, **caractérisé en ce que** ledit agent de coloration est utilisé en une quantité comprise entre 0,001 et 1 % en masse par rapport au volume total de solution $T_1$.

11. Procédé selon l'une quelconque des revendications précédentes, **caractérisé en ce que** ladite étape (b) comprend un (ou plusieurs) lavage(s) de la surface du support solide aux moyens de solutions de lavage identiques ou différentes.

12. Procédé selon la revendication 11, **caractérisé en ce que** lesdites solutions de lavage sont choisies dans le groupe constitué par l'eau, l'eau distillée, l'eau déminéralisée, l'eau désionisée, un tampon phosphate salin, une solution saline, un tampon acétate, un tampon carbonate, une solution aqueuse comprenant un alcool et/ou un acide telle que définie aux revendications 7 à 9 et leurs mélanges.

13. Procédé selon l'une quelconque des revendications 1 à 12, **caractérisé en ce que** ledit alcool dans la solution $T_2$ est choisi dans le groupe constitué par le méthanol, l'éthanol, le propanol, l'isopropanol, le butanol et leurs mélanges.

14. Procédé selon l'une quelconque des revendications précédentes, **caractérisé en ce que** lesdits ions carbonate dans la solution $T_2$ présentent une molarité comprise entre 0,001 et 1 M.

15. Utilisation d'un procédé tel que défini dans l'une quelconque des revendications 1 à **14** pour comparer différentes stratégies d'immobilisation de molécules comprenant au moins un groupement protoné sur des supports solides et susceptibles de générer des supports coatés, des puces à peptides, des puces à protéines, des puces à anticorps ou des puces à cellules.

16. Utilisation d'un procédé tel que défini dans l'une quelconque des revendications 1 à **14** pour vérifier la robustesse, la stabilité et la sensibilité des supports coatés, des puces à peptides, des puces à protéines, des puces à anticorps ou des puces à cellules.

17. Utilisation d'un procédé tel que défini dans l'une quelconque des revendications 1 à **14** pour détecter et éventuellement quantifier au moins une protéine, au moins un anticorps ou au moins un de leurs fragments présents dans un liquide d'intérêt.

18. Kit d'éléments pour la mise en oeuvre d'un procédé tel que défini dans l'une quelconque des revendications 1 à **14** contenant :

   - dans un premier compartiment, la solution $T_1$ telle que définie dans l'une quelconque des revendications 7 à 9 contenant du bleu de Coomassie G250 comme agent de coloration en une quantité supérieure ou égale à 0,05 % en masse par rapport au volume de la solution $T_1$ ;
   - dans un second compartiment, la solution $T_2$ telle que définie dans l'une quelconque des revendications 1, 13 et 14 ;
   - et, éventuellement, une (ou plusieurs) solution(s) de lavage telle(s) que définie(s) à la revendication 12.

19. Procédé selon l'une quelconque des revendications 1 à 15, **caractérisé en ce que ladite fonction amine** est choisi**e** dans le groupe constitué par une fonction amine primaire du type $-NH_2$, une fonction amine secondaire du type -NHR, R représentant un groupe carboné **et** une fonction amine tertiaire du type -NR'R, R et R' représentant des groupes carbonés identiques ou différents, ou appartenant à un même groupe carboné.

**Patentansprüche**

1. Verfahren zum Nachweis und zur Quantifizierung von mindestens einem Molekül, umfassend mindestens eine immobilisierte protonierte Gruppierung an der Oberfläche eines festen Trägers, wobei das Verfahren die folgenden sukzessiven Schritte umfasst:

   a) In-Kontakt-Bringen der Oberfläche des festen Trägers, auf der mindestens ein Molekül immobilisiert ist, umfassend mindestens eine protonierte Gruppierung mit einer Lösung $T_1$, enthaltend Coomassie-Blau G250 als Färbemittel,
   wobei die protonierte Gruppierung ausgewählt ist aus der Gruppe, bestehend aus einer Aminfunktion, einer Iminfunktion, einer Guanidinofunktion und einer Heteroarylgruppierung,
   b) Beseitigen des Färbungsmittels, das in Schritt (a) nicht mit der Oberfläche reagiert hat,
   c) In-Kontakt-Bringen der Oberfläche mit einer Lösung $T_2$, die ausgelegt ist, um den (oder die eventuellen) Komplex(e), gebildet zwischen dem Färbungsmittel und dem Molekül, umfassend mindestens eine protonierte Gruppierung, zu dissoziieren,
   wobei die Lösung $T_2$ eine wässrige Lösung ist, die Alkohol in einer Proportion im Bereich zwischen 40 und 60 Vol% mit Bezug auf das Gesamtvolumen $T_2$ der Carbonationen enthält,
   wobei der pH-Wert der Lösung $T_2$ höher als 11 ist,
   d) Versäuerung der Lösung $T_2$ und Nachweis des Färbungsmittels, das eventuell in der versäuerten Lösung $T_2$ enthalten ist, um das Molekül, umfassend mindestens eine protonierte Gruppierung, nachzuweisen und zu quantifizieren,

   wobei der Nachweis des Färbungsmittels bei Schritt (d) darin besteht, die optische Dichte der versäuerten Lösung T2, erhalten nach Schritt (c), zu messen,
   wobei der Nachweis und die Quantifizierung des Moleküls, umfassend mindestens eine protonierte Gruppierung, ausgehend von der reellen optischen Dichte erhalten werden, die der optischen Dichte der versäuerten Lösung $T_2$ entspricht, die nach Schritt (c) erhalten wird, von der die erhaltene optische Dichte unter den gleichen Bedingungen, jedoch bei Abwesenheit des mindestens einen Moleküls, umfassend mindestens eine protonierte Gruppierung, subtrahiert wurde.

2. Verfahren nach Anspruch 1, **dadurch gekennzeichnet, dass** das Molekül, umfassend mindestens eine protonierte Gruppierung, ausgewählt ist aus der Gruppe, bestehend aus Peptiden, Polypeptiden, Proteinen, Proteinen, die prosthetische Gruppen aufweisen, Antikörpern, transaminierten Nukleinsäuren, biogenen Aminen, Dendronen und Dendrimeren, ihren Fragmenten und ihren Derivaten.

3. Verfahren nach Anspruch 1 oder 2, **dadurch gekennzeichnet, dass** der feste Träger oder mindestens die Oberfläche des festen Trägers, wo das Molekül, umfassend mindestens eine protonierte Gruppierung, immobilisiert ist, ein fester Träger oder eine anorganische Oberfläche ist.

4. Verfahren nach Anspruch 1 oder 2, **dadurch gekennzeichnet, dass** der feste Träger oder mindestens die Oberfläche des festen Trägers, wo das Molekül, umfassend mindestens eine protonierte Gruppierung, immobilisiert ist, ein organisches Material ist.

5. Verfahren nach einem beliebigen der vorhergehenden Ansprüche, **dadurch gekennzeichnet, dass** der feste Träger eine Oberfläche aufweist, die funktionelle Gruppierungen trägt, dank derer das Molekül, umfassend mindestens eine protonierte Gruppe, dazu in der Lage ist, immobilisiert zu werden.

6. Verfahren nach einem beliebigen der vorhergehenden Ansprüche, **dadurch gekennzeichnet, dass** das Molekül, umfassend mindestens eine protonierte Gruppierung, auf indirekte Weise an der Oberfläche des festen Trägers immobilisiert ist.

7. Verfahren nach einem beliebigen der vorhergehenden Ansprüche, **dadurch gekennzeichnet, dass** die Lösung $T_1$ eine wässrige Lösung ist, umfassend einen Alkohol und/oder eine Säure, wobei der pH-Wert der Lösung $T_1$ vorzugsweise höher als 1 ist.

8. Verfahren nach Anspruch 7, **dadurch gekennzeichnet, dass** der Alkohol ausgewählt ist aus der Gruppe, bestehend aus Methanol, Ethanol, Propanol, Isopropanol, Butanol und ihren Mischungen, wobei die Alkoholproportion vorzugsweise im Bereich zwischen 1 und 25 Vol% mit Bezug auf das Gesamtvolumen der Lösung $T_1$ liegt.

9. Verfahren nach Anspruch 7 oder 8, **dadurch gekennzeichnet, dass** die Säure ausgewählt ist aus der Gruppe, bestehend aus Essigsäure, Trichloressigsäure und ihren Mischungen, wobei die Säureproportion vorzugsweise im Bereich zwischen 0,5 und 20 Vol% mit Bezug auf das Gesamtvolumen der Lösung $T_1$ liegt.

10. Verfahren nach einem beliebigen der vorhergehenden Ansprüche, **dadurch gekennzeichnet, dass** das Färbemittel in einer Menge verwendet wird, die im Bereich zwischen 0,001 und 1 Massenprozent mit Bezug auf das Gesamtvolumen der Lösung $T_1$ liegt.

11. Verfahren nach einem beliebigen der vorhergehenden Ansprüche, **dadurch gekennzeichnet, dass** der Schritt (b) eine (oder mehrere) Waschung(en) der Oberfläche des festen Trägers mit Hilfe von identischen oder verschiedenen Waschlösungen umfasst.

12. Verfahren nach Anspruch 11, **dadurch gekennzeichnet, dass** die Waschlösungen ausgewählt sind aus der Gruppe, bestehend aus Wasser, destilliertem Wasser, demineralisiertem Wasser, deionisiertem Wasser, einer phosphatgepufferten Salzlösung, einer Speichellösung, einem Acetatpuffer, einem Carbonatpuffer, einer wässrigen Lösung, umfassend einen Alkohol und/oder eine Säure, wie in Anspruch 7 bis 9 definiert, und ihren Mischungen.

13. Verfahren nach einem beliebigen der Ansprüche 1 bis 12, **dadurch gekennzeichnet, dass** der Alkohol in der Lösung $T_2$ ausgewählt ist aus der Gruppe, bestehend aus Methanol, Ethanol, Propanol, Isopropanol, Butanol und ihren Mischungen.

14. Verfahren nach einem beliebigen der vorhergehenden Ansprüche, **dadurch gekennzeichnet, dass** die Carbonationen in der Lösung $T_2$ eine Molarität aufweisen, die im Bereich zwischen 0,001 und 1 M liegt.

15. Verwendung eines Verfahrens, wie in einem beliebigen der Ansprüche 1 bis 14 definiert, um die verschiedenen Strategien der Immobilisierung von Molekülen zu vergleichen, umfassend mindestens eine protonierte Gruppierung auf festen Trägern und dazu geeignet, beschichtete Träger, Peptidchips, Proteinchips, Antikörperchips oder Zellchips zu generieren.

16. Verwendung eines Verfahrens, wie in einem beliebigen der Ansprüche 1 bis 14 definiert, um die Robustheit, die Stabilität und die Empfindlichkeit der beschichteten Träger, Peptidchips, Proteinchips, Antikörperchips oder Zellchips zu überprüfen.

17. Verwendung eines Verfahrens, wie in einem beliebigen der Ansprüche 1 bis 14 definiert, um mindestens ein Protein, mindestens einen Antikörper oder mindestens eines ihrer Fragmente, die in einer bestimmten Flüssigkeit vorhanden sind, nachzuweisen und eventuell zu klassifizieren.

18. Kit von Elementen zur Durchführung eines Verfahrens, wie in einem beliebigen der Ansprüche 1 bis 14 definiert, enthaltend:

   - in einem ersten Kompartiment die Lösung $T_1$, wie in einem beliebigen der Ansprüche 7 bis 9 definiert, enthaltend Coomassie-Blau G250 als Färbungsmittel in einer Menge von mehr als oder gleich wie 0,05 Massenprozent mit Bezug auf das Gesamtvolumen der Lösung $T_1$;
   - in einem zweiten Kompartiment die Lösung $T_2$, wie in einem beliebigen der Ansprüche 1, 13 und 14 definiert;
   - und eventuell eine (oder mehrere) Waschlösung(en), wie in Anspruch 12 definiert.

19. Verfahren nach einem beliebigen der Ansprüche 1 bis 15, **dadurch gekennzeichnet, dass** die Aminfunktion ausgewählt ist aus der Gruppe, bestehend aus einer primären Aminfunktion vom Typ $-NH_2$, einer sekundären Aminfunktion vom Typ -NHR, wobei R eine Kohlenstoffgruppe darstellt, und eine tertiäre Aminfunktion vom Typ -NR'R, wobei R und R' identische oder verschiedene Kohlenstoffgruppen darstellen oder zu einer gleichen Kohlenstoffgruppe gehören.

**Claims**

1. A method for detecting and quantifying at least one molecule comprising at least one proton group immobilized at the surface of a solid support, said method comprises the following successive steps of:

a) contacting said surface of the solid support on which is immobilized at least one molecule comprising at least one proton group with a solution $T_1$ containing Coomassie blue G250 as a coloring agent,

said proton group being selected from the group composed of an amine function, an imine function, a guanidino function and a heteroaryl group,

b) removing said coloring agent unreacted with said surface during step (a),

c) contacting said surface with a solution $T_2$ capable of dissociating the possible complex(es) formed between said coloring agent and said molecule comprising at least one proton group,

said solution $T_2$ being an aqueous solution containing an alcohol in a proportion comprised between 40% and 60% by volume relative to the total volume of the solution $T_2$ and carbonate ions,

the pH of said solution $T_2$ being greater than 11,

d) acidifying said solution $T_2$ and detecting said coloring agent possibly present in the acidified solution $T_2$ so as to detect and quantify said molecule comprising at least one proton group,

said detection of the coloring agent during step (d) consisting in measuring the optical density of the acidified solution $T_2$ obtained after step (c),

said detection and said quantification of said molecule comprising at least one proton group being obtained from the actual optical density corresponding to the optical density of the acidified solution $T_2$ obtained after step (c) from which the optical density, obtained under the same conditions but in the absence of said at least one molecule comprising at least one proton group, has been subtracted.

2. The method according to claim 1, **characterized in that** said molecule comprising at least one proton group is selected from the group composed of peptides, polypeptides, proteins, proteins having prosthetic groups, antibodies, transaminated nucleic acids, biogenic amines, dendrons and dendrimers, their fragments and derivatives.

3. The method according to claim 1 or 2, **characterized in that** said solid support or at least said surface of said solid support where said molecule comprising at least one proton group is immobilized, is an inorganic solid support or surface.

4. The method according to claim 1 or 2, **characterized in that** said solid support or at least said surface of said solid support where said molecule comprising at least one proton group is immobilized, is made of an organic material.

5. The method according to any one of the preceding claims, **characterized in that** said solid support has a surface carrying functional groups thanks to which said molecule comprising at least one proton group is able to be immobilized.

6. The method according to any one of the preceding claims, **characterized in that** said molecule comprising at least one proton group is indirectly immobilized at the surface of the solid support.

7. The method according to any one of the preceding claims, **characterized in that** said solution $T_1$ is an aqueous solution comprising an alcohol and/or an acid, the pH of said solution $T_1$ being, preferably, greater than 1.

8. The method according to claim 7, **characterized in that** said alcohol is selected from the group composed of methanol, ethanol, propanol, isopropanol, butanol and mixtures thereof, the proportion of alcohol being preferably comprised between 1 and 25% by volume relative to the total volume of the solution $T_1$.

9. The method according to claim 7 or 8, **characterized in that** said acid is selected from the group composed of acetic acid, trichloroacetic acid and mixtures thereof, the acid proportion being preferably comprised between 0.5 and 20% by volume relative to the total volume of the solution $T_1$.

10. The method according to any one of the preceding claims, **characterized in that** said coloring agent is used in an amount comprised between 0.001 and 1 weight % relative to the total volume of the solution $T_1$.

11. The method according to any one of the preceding claims, **characterized in that** said step (b) comprises one (or several) washing(s) of the surface of the solid support by means of identical or different washing solutions.

12. The method according to claim 11, **characterized in that** said washing solutions are selected from the group composed of water, distilled water, demineralized water, deionized water, a saline phosphate buffer, a saline solution, an acetate buffer, a carbonate buffer, an aqueous solution comprising an alcohol and/or an acid as defined in claims 7 to 9 and mixtures thereof.

**13.** The method according to any one of claims 1 to 12, **characterized in that** said alcohol in the solution $T_2$ is selected from the group composed of methanol, ethanol, propanol, isopropanol, butanol and mixtures thereof.

**14.** The method according to any one of the preceding claims, **characterized in that** said carbonate ions in the solution $T_2$ have a molarity comprised between 0.001 and 1 M.

**15.** A use of a method as defined in any one of claims 1 to 14, to compare different strategies for immobilizing molecules comprising at least one proton group on solid supports and likely to generate coated supports, peptide chips, protein chips, antibody chips or cell chips.

**16.** A use of a method as defined in any one of claims 1 to 14, to verify the robustness, stability, and sensitivity of coated supports, peptide chips, protein chips, antibody chips, or cell chips.

**17.** A use of a method as defined in any one of claims 1 to 14, to detect and possibly quantify at least one protein, at least one antibody or at least one of their fragments present in a liquid of interest.

**18.** A kit of elements for implementing a method as defined in any one of claims 1 to 14 containing:

- in a first compartment, the solution $T_1$ as defined in any one of claims 7 to 9 containing Coomassie blue G250 as a coloring agent in an amount greater than or equal to 0.05 weight % relative to the volume of the solution $T_1$ ;
- in a second compartment, the solution $T_2$ as defined in any one of claims 1, 13 and 14;
- and, optionally, one (or several) washing solution(s) as defined in claim 12.

**19.** The method according to any one of claims 1 to 15, **characterized in that** said amine function is selected from the group composed of a primary amine function of the -NH$_2$ type, a secondary amine function of the -NHR type, R representing a carbon group, and a tertiary amine function of the -NR'R type, R and R' representing identical or different carbon groups, or belonging to a same carbon group.

Bleu de coomassie
libre

Couche
d'amines
immobilisée

Support
solide

Etape (a)

Etape (b)

Support réutilisable

Détection
directe

Etape (c)

FIG.1

Etape (d)

**Droite d'étalonnage du bleu de Coomassie
dans le tampon de décoloration T$_2$/HCl**

Absorbance réelle

$y = 0,0802x + 0,0021$
$R^2 = 0,9995$

Conc du bleu en µg/mL

FIG.2A

test aux résidus réduits

FIG.2B

FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6696304 B
- US 6555382 B
- WO 2006114528 A

**Littérature non-brevet citée dans la description**

- **GOSNELL et al.** *Microchemical Journal,* 1988, vol. 37, 149-154
- **ORSCHEL et al.** *Colloids and Surfaces B : Biointerfaces,* 1998, vol. 10, 273-279
- *CHEMICAL ABSTRACTS,* 6226-79-5
- *CHEMICAL ABSTRACTS,* 3761-53-3
- *CHEMICAL ABSTRACTS,* 2611-82-7
- *CHEMICAL ABSTRACTS,* 2766-77-0
- *CHEMICAL ABSTRACTS,* 3567-69-9
- *CHEMICAL ABSTRACTS,* 17752-85-1
- *CHEMICAL ABSTRACTS,* 3844-45-9
- *CHEMICAL ABSTRACTS,* 2580-78-1
- *CHEMICAL ABSTRACTS,* 2353-45-9
- *CHEMICAL ABSTRACTS,* 1064-48-8
- *CHEMICAL ABSTRACTS,* 12236-82-7
- *CHEMICAL ABSTRACTS,* 910010-03-9
- *CHEMICAL ABSTRACTS,* 78642-64-5
- *CHEMICAL ABSTRACTS,* 6104-58-1
- *CHEMICAL ABSTRACTS,* 3301-79-9
- *CHEMICAL ABSTRACTS,* 17372-87-1
- *CHEMICAL ABSTRACTS,* 548-28-3
- *CHEMICAL ABSTRACTS,* 81-88-9
- *CHEMICAL ABSTRACTS,* 60311-02-6
- *CHEMICAL ABSTRACTS,* 6358-69-6
- *CHEMICAL ABSTRACTS,* 3599-32-4
- **ARKAS M. ; TSIOURVAS D . ; PALEOS C.M.** Organosilicon Dendritic Networks in Porous Ceramics for Water Purification. *Chemistry of Material,* 2005, vol. 17, 3439-44
- **ATHERTON, B. A. ; E.L. CUNNINGHAM ; A.G. SPLITTGERBER.** A mathematical model for the description of the coomassie brilliant blue protein assay. *Analytical Biochemistry,* 1996, vol. 233, 160-168
- **BRADFORD, M. M.** A rapid and sensitive method for the quantitation of microgram quantifies of protein utilizing the principle of protein-dye binding. *Analytical Biochemistry,* 1976, vol. 72, 248-254
- **CHIAL, H. J. ; H. B. THOMPSON et al.** A spectral study of the charge forms of Coomassie blue G. *Analytical Biochemistry,* 1993, vol. 209 (2), 258-266
- **CHIAL, H. J. ; A. G. SPLITTGERBER.** A comparison of the binding of Coomassie brilliant blue to proteins at low and neutral pH. *Analytical Biochemistry,* 1993, vol. 213 (2), 362-369
- **CONGDON, R. W. ; G. W. MUTH et al.** The binding interaction of Coomassie blue with proteins. *Analytical Biochemistry,* 1993, vol. 213 (2), 407-413
- **CLAEYS-BRUNO, M. ; O. VANDENABEELE-TRAMBOUZE et al.** Methodological approaches for histamine quantification using derivatization by chloroethylnitrosourea and ELISA measurement. Part I. Optimization of derived histamine detection with coated-plates using optimal design and Part II: Optimisation of the derivatization step. *Chemometrics and Intelligent Laboratory Systems,* 2006, vol. 80 (2), 176-197
- **METWALLI E. ; HAINES D. BECKER O. ; CONZONE S. ; PANTANO C.G.** Surface characterizations of mono-, di-, and tri-aminosilane treated glass substrates. *Journal of Colloid and Interface Science,* 2006, vol. 298, 8252-31
- **PATHAK S. ; SINGH A.K. ; MCELHANON J.R. ; DENTINGER P.M.** Dendrimer-activated surfaces for high density and high activity protein chip applications. *Langmuir,* 2004, vol. 20, 6075-79